(19) **Europäisches Patentamt**

**European Patent Office**

**Office européen des brevets**

(11) **EP 1 734 030 A1**

(12) **EUROPÄISCHE PATENTANMELDUNG**

(43) Veröffentlichungstag:
20.12.2006 Patentblatt 2006/51

(51) Int Cl.:
*C07C 57/05* (2006.01)

(21) Anmeldenummer: 06100535.1

(22) Anmeldetag: 18.01.2006

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **BASF Aktiengesellschaft 67056 Ludwigshafen (DE)**

(72) Erfinder:
 • **Cremer, Ulrich.**
  **68161 Mannheim (DE)**
 • **Dieterle, Martin.**
  **68167 Mannheim (DE)**
 • **Müller-Engel, Klaus Joachim.**
  **76297 Stutensee (DE)**

(54) **Verfahren zum Langzeitbetrieb einer heterogen katalysierten partiellen Gasphasenoxidation einer organischen Ausgangsverbindung**

(57) Verfahren zum Langzeitbetrieb einer heterogen katalysierten partiellen Gasphasenoxidation einer organischen Ausgangsverbindung, bei dem man das Reaktionsgaseingangsgemisch an einem Katalysatorfestbett partialoxidiert, das in zwei aufeinanderfolgenden Temperaturzonen A, B untergebracht ist, deren Temperatur mit zunehmender Betriebsdauer so verändert wird, dass sich die anfangs tiefere Temperatur erhöht und der Unterschied zwischen den beiden Temperaturen verringert.

EP 1 734 030 A1

**Beschreibung**

Verfahren zum Langzeitbetrieb einer heterogen katalysierten partiellen Gasphasenoxidation (bzw. Gasphasen-Partialoxidation) einer organischen Ausgangsverbindung zu einer organischen Zielverbindung, bei dem man ein die organische Ausgangsverbindung und molekularen Sauerstoff enthaltendes Reaktionsgaseingangsgemisch zunächst ("anfangs") mit der Maßgabe durch ein frisch beschicktes Katalysatorfestbett, das in zwei räumlich aufeinanderfolgenden (und in der Regel aneinandergrenzenden) Temperaturzonen A, B aufgeschüttet ist, deren Temperaturen $T^A$ und $T^B$ so beschaffen sind, dass die Differenz $\Delta T^{BA}$ zwischen der Temperatur $T^B$ der Temperaturzone B und der Temperatur $T^A$ der Temperaturzone A und berechnet mit der höheren der beiden Temperaturen als Minuend (d.h., die tiefere der beiden Temperaturen $T^B$, $T^A$ ist der Subtrahend) > 0 °C beträgt, führt, dass das Reaktionsgaseingangsgemisch die Temperaturzonen A, B in der zeitlichen Abfolge "erst A" und "dann B" durchströmt, wobei sich die Temperaturzone A bis zu einem Umsatz der organischen Ausgangsverbindung von $U^A$ = 15 bis 85 mol-% erstreckt und sich in der Temperaturzone B der Umsatz der organischen Ausgangsverbindung auf einen Wert $U^B \geq$ 90 mol-% erhöht und bei dem man anschließend mit zunehmender Betriebsdauer, um der Minderung der Qualität des Katalysatorfestbetts entgegenzuwirken, die Temperatur der Temperaturzonen A, B verändert. Ist $T^B$ der Minuend, so ist $T^A$ der Subtrahend und die Differenzbildung zur Bestimmung von $\Delta T^{BA}$ wie folgt vorzunehmen: $\Delta T^{BA} = T^B\text{-}T^A$.

Unter einer vollständigen Oxidation einer organischen Verbindung mit molekularem Sauerstoff wird hier verstanden, dass die organische Verbindung unter reaktiver Einwirkung von molekularem Sauerstoff so umgesetzt wird, dass der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird. Alle davon verschiedenen Umsetzungen einer organischen Verbindung unter reaktiver Einwirkung von molekularem Sauerstoff werden hier als Partialoxidationen einer organischen Verbindung zusammengefasst.

Im besonderen sollen hier unter Partialoxidationen solche Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält als vor Durchführung der Partialoxidation.

Als ein sich unter den Bedingungen der heterogen katalysierten Gasphasen-Partialoxidation im wesentlichen inert verhaltendes Verdünnungsgas werden solche Verdünnungsgase verstanden, deren Bestandteile unter den Bedingungen der heterogen katalysierten Gasphasen-Partialoxidation-jeder Bestandteil für sich betrachtet - zu mehr als 95 mol-%, vorzugsweise zu mehr als 99 mol-% unverändert erhalten bleiben.

Unter der Belastung eines einen Reaktionsschritt katalysierenden Katalysatorfestbetts mit Reaktionsgasgemisch wird die Menge an Reaktionsgasgemisch in Normlitern (= NI; das Volumen in Litern, das die entsprechende Reaktionsgasgemischmenge bei Normalbedingungen, d. h., bei 0 °C und 1 atm, einnehmen würde) verstanden, die dem Katalysatorfestbett, bezogen auf das Volumen seiner Schüttung (reine Inertmaterialabschnitte werden dabei nicht miteinbezogen), pro Stunde zugeführt wird ($\rightarrow$ Einheit = NI/I•h). Die Belastung kann auch nur auf einen Bestandteil des Reaktionsgasgemischs bezogen sein. Dann ist es die Volumenmenge dieses Bestandteils, die dem Katalysatorfestbett, bezogen auf das Volumen seiner Schüttung, pro Stunde zugeführt wird.

Es ist allgemein bekannt, dass durch partielle und heterogen katalysierte Oxidation verschiedenster organischer Ausgangsverbindungen mit molekularem Sauerstoff in der Gasphase im Katalysatorfestbett zahlreiche Grundchemikalien (Zielprodukte) erzeugt werden können. Beispielhaft genannt seien die Umsetzung von Propylen zu Acrolein und/oder Acrylsäure (vgl. z. B. DE-A 23 51 151), die Umsetzung von tert.-Butanol, iso-Buten, iso-Butan, iso-Butyraldehyd oder dem Methylether des tert.-Butanols zu Metacrolein und/oder Methacrylsäure (vgl. z. B. DE-A 25 26 238, EP-A 092 097, EP-A 058 927, DE-A 41 32 263, DE-A 41 32 684 und DE-A 40 22 212), die Umsetzung von Acrolein zu Acrylsäure, die Umsetzung von Methacrolein zu Methacrylsäure (vgl. z. B. die DE-A 25 26 238), die Umsetzung von o-Xylol, p-Xylol oder Naphtalin zu Phthalsäureanhydrid (vgl. z. B. EP-A 522 871) oder den entsprechenden Säuren sowie die Umsetzung von Butadien zu Maleinsäureanhydrid (vgl. z. B. GB-A 1 464 198 und GB-A 1 291 354), die Umsetzung von Indanen zu z. B. Anthrachinon (vgl. z. B. DE-A 20 25 430), die Umsetzung von Ethylen zu Ethylenoxid oder von Propylen zu Propylenoxid (vgl. z. B. DE-AS 12 54 137, DE-A 21 59 346, EP-A 372 972, WO 89/07101, DE-A 43 11 608 und Beyer, Lehrbuch der organischen Chemie, 17. Auflage (1973), Hirzel Verlag Stuttgart, Seite 261), die Umsetzung von Propylen und/oder Acrolein zu Acrylnitril (vgl. z. B. die DE-A 23 51 151), die Umsetzung von iso-Buten und/oder Methacrolein zu Methacrylnitril (d. h., der Begriff der partiellen Oxidation soll in dieser Schrift auch die partielle Ammoxidation, d. h., eine partielle Oxidation im Beisein von Ammoniak umfassen), die oxidative Dehydrierung von Kohlenwasserstoffen (vgl. z. B. die DE-A 23 51 151), die Umsetzung von Propan zu Acrylnitril oder zu Acrolein und/oder Acrylsäure (vgl. z. B. DE-A 101 31 297, EP-A 1 090 684, EP-A 608 838, DE-A 100 46 672, EP-A 529 853, WO 01/96270 und DE-A 100 28 582), die Umsetzung von iso-Butan zu Methacrolein und/oder Methacrylsäure, sowie die Reaktionen von Ethan zu Essigsäure, von Ethylen zu Ethylenoxid, von Benzol zu Phenol sowie von 1-Buten oder 2-Buten zu den entsprechenden Butandiolen etc..

Dem Katalysatorfestbett kommt dabei die Aufgabe zu, zu bewirken, dass die gewünschte Gasphasen-Partia-

Ioxidation gegenüber der vollständigen Oxidation bevorzugt abläuft.

Die chemische Umsetzung erfolgt, wenn das Reaktionsgasgemisch das Festbett durchströmt während der Verweilzeit des Reaktionsgasgemisches in selbigem.

Bei den Festkörperkatalysatoren handelt es sich häufig um Oxidmassen oder um Edelmetalle (z. B. Ag). Die katalytisch aktive Oxidmasse kann neben Sauerstoff lediglich ein anderes Element oder mehr als ein anderes Element (im Fall von sogenannten Multielementoxidmassen) enthalten.

Besonders häufig kommen als katalytisch aktive Oxidmassen solche zur Anwendung, die mehr als eine metallisches, insbesondere übergangsmetallisches, Element umfassen. In diesem Fall spricht man von Multimetalloxidmassen. Üblicherweise sind diese keine einfachen physikalischen Gemische von Oxiden ihrer elementaren Konstituenten, sondern Gemische von komplexen Polyverbindungen dieser Elemente. In der Praxis werden die vorgenannten katalytisch aktiven Festmassen in der Regel zu unterschiedlichsten Geometrien geformt (Ringe, Vollzylinder, Kugeln etc.) eingesetzt. Die Formgebung (zum Formkörper) kann dabei so erfolgen, dass die katalytisch aktive Masse als solche (z. B. in Extrudern oder Tablettiervorrichtungen) geformt wird, so dass im Ergebnis ein sogenannter Vollkatalysator resultiert, oder dadurch, dass die Aktivmasse auf einen vorgeformten Träger aufgebracht wird (vgl. z. B. WO 2004/009525 und WO 2005/113127).

Beispiele für Katalysatoren, die für erfindungsgemäße heterogen katalysierte Festbett-Gasphasen-Partialoxidationen wenigstens einer organischen Ausgangsverbindung geeignet sind, finden sich z. B. in der DE-A 100 46 957, in der EP-A 1 097 745, in der DE-A 44 31 957, in der DE-A 100 46 928, in der DE-A 199 10 506, in der DE-A 196 22 331, in der DE-A 101 21 592, in der EP-A 700 714, in der DE-A 199 10 508, in der EP-A 415 347, in der EP-A 471 853 und in der EP-A 700 893.

Üblicherweise verlaufen heterogen katalysierte Gasphasen-Partialoxidationen stark exotherm. Infolge einer Vielfalt möglicher Parallel- und/oder Folgereaktionen ist im Hinblick auf eine möglichst selektive Umsetzung der partiell zu oxidierenden wenigstens einen organischen Ausgangsverbindung zum gewünschten Zielprodukt die alleinige Maßnahme einer Katalysatormitverwendung normalerweise nicht ausreichend. Vielmehr ist es für eine möglichst selektive Durchführung einer heterogen katalysierten Gasphasen-Partialoxidation im Katalysatorfestbett zusätzlich erforderlich, den Verlauf der Reaktionstemperatur bzw. den Verlauf der Temperatur des Katalysatorfestbetts in Strömungsrichtung des Reaktionsgasgemischs in einem gewissen Umfang zu lenken.

Gemäß den Lehren des Standes der Technik hat es sich diesbezüglich in der Regel als vorteilhaft erwiesen, ein frisch beschicktes Katalysatorfestbett in zwei räumlich aufeinanderfolgenden Temperaturzonen A, B aufzuschütten, deren Temperaturen $T^A$ und $T^B$ so beschaffen sind, dass die Differenz $\Delta T^{BA}$ zwischen der Temperatur $T^B$ der Temperaturzone B und der Temperatur $T^A$ der Temperaturzone A und berechnet mit der höheren der beiden Temperaturen als Minuend > 0 °C beträgt, und das die organische Ausgangsverbindung und den molekularen Sauerstoff enthaltende Reaktionsgaseingangsgemisch so durch das Katalysatorfestbett zu führen, dass das Reaktionsgaseingangsgemisch die Temperaturzonen A, B in der zeitlichen Abfolge "erst A" und "dann B" durchströmt, wobei die Länge der Temperaturzone A so bemessen ist, dass sie sich bis zu einem Umsatz der organischen Ausgangsverbindung von $U^A$= 15 bis 85 mol-% erstreckt und die Länge der Temperaturzone B so bemessen ist, dass sich in der Temperaturzone **B** der Umsatz der organischen Ausgangsverbindung auf einen Wert $U^B \geq 90$ mol-% erhöht (vgl. z.B. DE-A 199 27 624, DE-A 199 48 523, WO 00/53557, DE-A 199 48 248, WO 00/53558, WO 2004/085365, WO 2004/085363, WO 2004/085367, WO 2004/085369, WO 2004/085370, WO 2004/085362, EP-A 1 159 247, EP-A 1 159 246, EP-A 1 159 248, EP-A 1 106 598, WO 2005/021149, US-A 2005/0049435, WO 2004/007064, WO 05/063673, WO 05/063674).

In der Praxis werden die Temperaturzonen A, B dabei in der Regel so verwirklicht, dass man das Katalysatorfestbett in einen Reaktionsraum einbringt (in einem Reaktionsraum aufschüttet), um den in zwei voneinander im wesentlichen getrennten, in Strömungsrichtung des Reaktionsgasgemischs räumlich aufeinanderfolgenden (und in der Regel aneinandergrenzenden) Abschnitten A, B aus Gründen der Wärmeabfuhr jeweils ein fluider (vorzugsweise flüssiger) Wärmeträger (ein Wärmeaustauschmittel) geführt bzw. geleitet (ein- und aus-) wird, der die materielle Einhüllende des Reaktionsraums (die Wand des Reaktionsraums) entlang des jeweiligen Abschnitts A bzw. B berührt (sich mit selbiger in Kontakt befindet). Der im Abschnitt A geführte Wärmeträger wird dabei normalerweise mit der Temperatur $T^A$ und der im Abschnitt B geführte Wärmeträger wird dabei normalerweise mit der Temperatur $T^B$ zugeführt. Die Gesamtkapazität des geführten Wärmeträgerstroms ist dabei normalerweise sehr viel größer als die Gesamtwärmekapazität des geführten Reaktionsgasgemischstroms.

Unter der Temperatur einer Temperaturzone wird dabei im vorstehend zitierten Stand der Technik, wie auch in dieser Schrift, die Temperatur des in der Temperaturzone befindlichen Teils der Festbettkatalysatorschüttung (des Katalysatorfestbetts) bei Ausübung des erfindungsgemäßen Verfahrens, jedoch in fiktiver Abwesenheit der chemischen Reaktionswärme verstanden.

Beispielsweise und anwendungstechnisch besonders einfach kann sich das Katalysatorfestbett in den Kontaktrohren (Reaktionsrohren) eines sogenannten Zweizonenrohrbündelreaktors befinden (aufgeschüttet sein), wie er z. B. in den DE-A's 199 10 508, 199 48 523, 199 10 506 und 199 48 241 sowie in den Schriften WO 2004/085362, WO 2004/085370, WO 2004/085369, WO 2004/085363, WO 2004/085365, WO 2004/007064 und WO 2004/085367 beschrieben ist. Eine bevorzugte Variante eines erfindungsgemäß einsetzbaren Zweizonenrohrbündelreaktors offenbart die DE-C 28 30 765. Aber auch die in der DE-C 25 13 405, der US-A 3,147,084, der DE-A 22 01 528, der EP-A 383 224 und der DE-A 29 03 218 offenbarten Zweizonenrohrbündelreaktoren sind für eine Durchführung des erfindungsgemäßen Verfahrens geeignet.

D.h., in einfachster Weise befindet sich die erfindungsgemäß zu verwendende Festbettkatalysatorschüttung in den Reaktionsrohren eines Vielkontaktrohr-Festbettreaktors (Rohrbündelreaktors) und um die Reaktionsrohre werden zwei voneinander im wesentlichen räumlich getrennte Temperiermedien (z. B. ionische Flüssigkeiten, Wasser (Dampf), Salzschmelzen oder flüssige Metalle) geführt (geleitet; ein- und aus-). Der Rohrabschnitt, über den sich das jeweilige Salzbad bzw. Metallbad erstreckt, repräsentiert eine Temperaturzone.

Zusätzlich zu den vorstehend beschriebenen externen Maßnahmen der Temperaturlenkung werden die Reaktionspartner üblicherweise mit einem sich unter den Bedingungen der heterogen katalysierten Gasphasen-Partialoxidation im wesentlichen inerten Gas verdünnt, das mit seiner Wärmekapazität frei werdende Reaktionswärme zu absorbieren vermag (interne Maßnahme der Temperaturlenkung).

Das Reaktionsgasgemisch einer wie eingangs beschriebenen heterogen katalysierten Gasphasen-Partialoxidation wenigstens einer organischen Ausgangsverbindung wird daher neben der wenigstens einen organischen Ausgangsverbindung und molekularem Sauerstoff in der Regel zusätzlich wenigstens ein inertes Verdünnungsgas umfassen.

Eines der häufigsten mitverwendeten inerten Verdünnungsgase ist molekularer Stickstoff, der automatisch immer dann zur Anwendung kommt, wenn als Sauerstoffquelle für die heterogen katalysierte Gasphasen-Partialoxidation Luft verwendet wird.

Ein anderes vielfach mitverwendetes inertes Verdünnungsgas ist wegen seiner allgemeinen Verfügbarkeit und vorteilhaften spezifischen Wärme Wasserdampf.

Andere in typischer Weise mitverwendete inerte Verdünnungsgase sind Edelgase (z. B. He, Ar, Ne) oder die Kohlenoxide $CO_2$ und/oder $CO$.

Die Verwendung von Verdünnungsgasen mit möglichst großer molarer Wärmekapazität ist üblicherweise besonders vorteilhaft (vgl. z. B. EP-A 253 409). Dazu zählen z. B. im Fall einer Partialoxidation einer ungesättigten organischen Ausgangsverbindung u. a. häufig gesättigte Kohlenwasserstoffe wie z. B. Propan im Fall einer Partialoxidation von Propylen zu Acrolein und/oder Acrylsäure.

Vielfach wird auch Kreisgas als inertes Verdünnungsgas mitverwendet (vgl. EP-A 1 180 508). Als Kreisgas wird das Restgas bezeichnet, das nach einer einstufigen oder mehrstufigen (die Mehrstufigkeit wird in der Regel dann angewandt, wenn sich die Partialoxidation einer organische Ausgangsverbindung zu einer Zielverbindung in aufeinanderfolgenden Schritten vollzieht; in diesen Fällen ist es häufig zweckmäßig, sowohl das Katalysatorfestbett als auch die sonstigen Reaktionsbedingungen an den jeweiligen Reaktionsschritt optimierend anzupassen und den jeweiligen Reaktionsschritt an einem diesen Reaktionsschritt spezifisch (maßgeschneidert) katalysierenden Katalysatorfestbett, das häufig in einem räumlich getrennten Reaktor bzw. in einem räumlich getrennten Reaktionsabschnitt aufgeschüttet (untergebracht) ist, d.h., als eine bzw. in einer separaten Reaktionsstufe durchzuführen; sie kann aber auch dann angewandt werden, wenn aus Gründen der Wärmeabfuhr oder aus anderen Gründen (vgl. DE-A 199 02 562) der Umsatz auf mehrere hintereinandergeschaltete Reaktoren verschmiert wird; ein Beispiel für eine häufig zweistufig durchgeführte heterogen katalysierte Gasphasen-Partialoxidation ist die Partialoxidation von Propylen zu Acrylsäure; in der ersten Reaktionsstufe wird das Propylen zum Acrolein und in der zweiten Reaktionsstufe Acrolein zur Acrylsäure partialoxidiert; in entsprechender Weise wird häufig auch die Methacrylsäureherstellung, meist ausgehend von iso-Buten, zweistufig durchgeführt; beide vorgenannten Partialoxidationen können bei Verwendung geeigneter Katalysatorbeschickungen aber auch einstufig (beide Schritte an einem in einem Reaktor aufgeschütteten (untergebrachten) Katalysatorfestbett mit beide Schritte katalysierendem Katalysator) durchgeführt werden, wie es z. B. für die Partialoxidation von Propylen zu Acrylsäure in der DE-A 101 21 592 beschrieben ist; bei der mehrstufigen Partialoxidation wird in der Regel das Produktgasgemisch der vorhergehenden Stufe ohne Zwischenproduktabtrennung, gegebenenfalls nach Zusatz von Inertgas und/oder molekularem Sauerstoff als Sekundärgas sowie gegebenenfalls nach erfolgter direkter und/oder indirekter Abkühlung als solches zur Beschickung der nachfolgenden Reaktionsstufe verwendet) heterogen katalysierten Gasphasen-Partialoxidation wenigstens einer organischen Verbindung dann verbleibt, wenn man aus dem Produktgasgemisch das Zielprodukt mehr oder weniger selektiv (z. B. durch Absorption in ein geeignetes Lösungsmittel oder durch fraktionierende Kondensation oder durch eine Überlagerung von Absorption und Kondensation) abgetrennt hat.

Im Regelfall besteht es überwiegend aus den für die Partialoxidation verwendeten inerten Verdünnungsgasen sowie aus bei der Partialoxidation üblicherweise als Nebenprodukt gebildetem oder als Verdünnungsgas zugesetztem Wasserdampf und durch unerwünschte vollständige Oxidation als Nebenreaktion gebildeten Kohlenoxiden. Teilweise enthält es noch geringe Mengen an bei der Partialoxidation nicht verbrauchtem molekularem Sauerstoff (Restsauerstoff) und/oder an nicht umgesetzter organischer Ausgangsverbindung und/oder nicht umgesetztem Zwischenprodukt.

Die mitverwendeten inerten Verdünnungsgase sind aber nicht nur dabei behilflich, die Reaktionswärme aufzunehmen, sondern gewährleisten in der Regel gleichzeitig einen sicheren Betrieb der heterogen katalysierten Gasphasen-Partialoxidation der organischen Ausgangsverbindung, indem sie das Reaktionsgasgemisch entweder außerhalb des Explosionsbereichs oder in einer noch sicher beherrschbaren Region des explosiven Bereichs halten.

Trotz der beschriebenen externen und internen Maßnahmen zur Lenkung (Steuerung) der Reaktionstemperatur bzw. der Temperatur des Katalysatorfestbetts sind die Temperaturen der Temperaturzonen A, B normalerweise von der in Strömungsrichtung längs des Katalysatorfestbetts jeweils herrschenden Reaktionstemperatur (= die jeweils herrschende Temperatur des Reaktionsgasgemischs) bzw. jeweils vorliegenden effektiven Temperatur des Katalysatorfestbetts (sie entspricht im wesentlichen der auf gleicher Höhe vorliegenden Reaktionstemperatur) verschieden. Die effektive Temperatur des Katalysatorfestbetts ist dabei die tatsächliche Temperatur des Katalysatorfestbetts, die sowohl den Einfluss des außerhalb des Reaktionsraums geführten fluiden Wärmeträgers, als auch die Reaktionswärme der Partialoxidation beinhaltet (wohingegen der Begriff "Temperatur der Temperaturzone", wie bereits gesagt, den Einfluss der Reaktionswärme der Partialoxidation ausklammert). Die Temperatur einer Temperaturzone ist im Unterschied zur effektiven Temperatur des Katalysatorfestbetts in Strömungsrichtung längs desselben normalerweise im wesentlichen konstant. Ist die Temperatur einer Temperaturzone nicht völlig konstant, so meint der Begriff Temperatur einer Temperaturzone hier den (Zahlen)mittelwert der Temperatur über die Temperaturzone. Die Temperierung der einzelnen Temperaturzonen erfolgt im wesentlichen unabhängig voneinander. Normalerweise ist die effektive Temperatur des Katalysatorfestbetts auf der jeweiligen Betthöhe größer als die Temperatur der zugehörigen Temperaturzone.

Von Bedeutung im vorgenannten Zusammenhang ist, dass die Temperatur des Reaktionsgasgemischs (und damit auch die effektive Temperatur des Katalysatorfestbetts) beim Durchschreiten des Katalysatorfestbetts in Strömungsrichtung des Reaktionsgasgemischs in der jeweiligen Temperaturzone üblicherweise einen Höchstwert durchläuft bzw. von einem solchen Maximalwert ausgehend abfällt (der sogenannte Heißpunktwert $T^{maxA}$ (in der **T**emperaturzone A) bzw. $T^{maxB}$ (in der **T**emperaturzone B)). Die Differenz zwischen Heißpunktwert und der Temperatur der zugehörigen Temperaturzone wird als Heißpunktausdehnung $\Delta T^{HB}_A$ (in der Temperaturzone A) bzw. $\Delta T^{HB}_B$ (in der Temperaturzone B) bezeichnet.

Eine Ursache dafür ist, dass die Reaktantenkonzentration im Reaktionsgasgemisch am Eingang (Eintritt) des Reaktionsgasgemischs in das Katalysatorfestbett am höchsten ist, was dort besonders hohe Reaktionsgeschwindigkeiten bedingt, mit denen pro Zeiteinheit besonders hohe Reaktionswärmeentwicklung einhergeht (beim Eintritt in das Katalysatorfestbett weist das Reaktionsgasgemisch (= das Reaktionsgaseingangsgemisch) in der Regel im wesentlichen die Temperatur der Temperaturzone A auf).

Eine andere Ursache dafür ist der endliche Wärmeübergang vom Reaktionsgasgemisch auf den Wärmeträger.

Gemäß der Lehre des Standes der Technik werden beim frisch beschickten Katalysatorfestbett die allgemeinen Verfahrensbedingungen in der Regel vorteilhaft so gewählt, dass $T^{maxA} - T^{maxB} \geq 0$ °C beträgt (vgl. WO 2004/085362, WO 2004/085370 und WO 2004/085363).

Ferner werden gemäß den Lehren des zitierten Standes der Technik beim frisch beschickten Katalysatorfestbett die allgemeinen Verfahrensbedingungen normalerweise so gewählt, dass sowohl $\Delta T^{HB}_B$ als auch $\Delta T^{HB}_A$ in der Regel 80 °C nicht überschreiten. Meist betragen diese Temperaturunterschiede $\leq 70$ °C, häufig 20 bis 70 °C und vorzugsweise sind die Temperaturunterschiede gering.

Weiterhin beträgt beim frisch beschickten Katalysatorfestbett (vorzugsweise gleichzeitig) die Änderung von $\Delta T^{HB}_A$ bzw. $\Delta T^{HB}_B$ bei Erhöhung der Temperatur der zugehörigen Temperaturzone um + 1 °C normalerweise (vgl. die gewürdigten Schriften des Standes der Technik) $\leq 9$ °C, vorzugsweise $\leq 7$ °C, oder $\leq 5$ °C bzw. $\leq 3$ °C.

Meist benötigen heterogen katalysierte Gasphasen-Partialoxidationen für auf einen einmaligen Durchgang des Reaktionsgasgemischs durch das Katalysatorfestbett bezogene wirtschaftliche Eduktumsätze der Partialoxidation erhöhte Temperaturen in den Temperaturzonen A, B. In der Regel betragen diese einige hundert °C, in typischer Weise 100 bis 600 °C, häufig 150 bis 500 °C, meist 200 bzw. 250 bis 450°C.

Der Arbeitsdruck kann bei heterogen katalysierten Gasphasen-Partialoxidationen am Katalysatorfestbett unter 1 atm oder über 1 atm liegen. In der Regel liegt er im Bereich von $\geq 1$ bis 20, bzw. bis 10 atm. Ein Arbeitsdruck von 100 atm wird üblicherweise nicht überschritten.

Es ist allgemein bekannt, dass heterogen katalysierte Gasphasen-Partialoxidationen einer organischen Ausgangsverbindung zu einer organischen Zielverbindung (zu einem Zielprodukt), bei denen man ein die organische Ausgangsverbindung und molekularen Sauerstoff enthaltendes Reaktionsgaseingangsgemisch mit der Maßgabe durch ein frisch beschicktes Katalysatorfestbett, das in zwei räumlich aufeinanderfolgenden Temperaturzonen A, B aufgeschüttet ist, deren Temperaturen $T^A$ und $T^B$ so beschaffen sind, das die Differenz $\Delta T^{BA}$ zwischen der Temperatur $T^B$ der Temperaturzone B und der Temperatur $T^A$ der Temperaturzone A und berechnet mit der höheren der beiden Temperaturen als Minuend > 0 °C beträgt, führt, dass das Reaktionsgaseingangsgemisch die Temperaturen A, B in der zeitlichen Abfolge "erst A" und "dann B" durchströmt, wobei sich die Temperaturzone A bis zu einem Umsatz der organischen Ausgangsverbindung von $U^A$ = 15 bis 85 mol-% erstreckt und sich in der Temperaturzone B der Umsatz der organischen Ausgangsverbindung auf einen Wert $U^B = \geq 90$ mol-% erhöht, im wesentlichen kontinuierlich über einen längeren Zeitraum unter im wesentlichen unveränderten Bedingungen an ein und demselben Katalysatorfestbett betrieben werden können.

Allerdings verliert das Katalysatorfestbett dabei mit zunehmender Betriebszeit normalerweise an Qualität. In der Regel verschlechtert sich vor allem die volumenspezifische Aktivität des Katalysatorfestbetts (unter ansonsten unveränderten Verfahrensbedingungen nimmt der auf den einmaligen Durchgang des Reaktionsgasgemischs durch das Katalysatorfestbett bezogene Eduktumsatz mit zunehmender Betriebszeit ab, was die beabsichtigte Raum-Zeit-Ausbeute einer Produktionsanlage an Zielprodukt mindert). Meist leidet auch die Selektivität der Zielproduktbildung.

Die EP-A 1 106 598 und die DE-A 10351269 versuchen der vorgenannten Entwicklung im Langzeitbetrieb einer wie beschrieben vorteilhaft durchzuführenden heterogen katalysierten Gasphasen-Partialoxidation einer organischen Ausgangsverbindung an ein und demselben Katalysatorfestbett dadurch Rechnung zu tragen, dass die Temperatur des Katalysatorfestbetts im Verlauf der Betriebszeit unter ansonsten weitgehend gleichbleibenden Betriebsbedingungen nach und nach erhöht wird, um den Eduktumsatz bei einmaligem Durchgang des Reaktionsgasgemischs durch das Katalysatorfestbett im wesentlichen beizubehalten (dabei kann, wie z.B. die WO 2004/085369, die DE-A 103 51 269, die DE-A 103 50 812, die DE-A 103 50 822 und die EP-A 614 872 empfehlen, der Qualitätsminderung des Katalysatorfestbetts im Langzeitbetrieb zwischendurch zusätzlich dadurch entgegengetreten werden, dass das Katalysatorfestbett von Zeit zu Zeit regeneriert wird; dazu wird das Verfahren der heterogen katalysierten Festbett-Gasphasen-Partialoxidation unterbrochen (z.B. dann, wenn im Aufarbeitungsteil einer (Meth)acrylsäureanlage in unerwünschter Weise gebildetes (Meth)acrylsäurepolymerisat zu entfernen und in diesem Zusammenhang auch die Partialoxidation zu unterbrechen ist, oder dann, wenn die Partialoxidation deshalb unterbrochen wird, weil das Reaktionsgasgemisch versehentlich eine explosionstechnisch gegebenenfalls nur schwer beherrschbare Zusammensetzung angenommen hat) und z.B. ein heißes Gemisch aus molekularem Sauerstoff und Inertgas durch das Katalysatorfestbett geführt). Eine solche Regenerierung kann auch gemäß der DE-A 102004008573, bzw. der WO 05/082517 erfolgen.

Nachteilig an den Lehren der EP-A 1 106 598 und der DE-A 10351269 ist jedoch, dass sie eine synchrone Erhöhung der Temperatur in den beiden Temperaturzonen A, B nahe legen. D. h., $T^A$ und $T^B$ werden im gleichen Ausmaß (um gleich viel °C) erhöht.

Eine solche Verfahrensweise ist zwar gegenüber einer Verfahrensweise ohne Erhöhung der Temperatur des Katalysatorfestbetts vorteilhaft und kann auch grundsätzlich bei allen in dieser Schrift angesprochenen Partialoxidationsverfahren vorteilhaft angewendet werden (z. B. insbesondere auf die in den Schriften WO 2004/085362, WO 2004/085370, WO 2004/085369, WO 2004/085363, WO 2004/085365 und WO 2004/085367 beschriebenen Verfahren der Partialoxidation von Propylen zu Acrolein sowie von Acrolein zu Acrylsäure).

Sie ist jedoch insofern nachteilig, als sie zwar unter ansonsten unveränderten Betriebsbedingungen den Beibehalt des angestrebten Eduktumsatzes (bezogen auf einmaligen Durchgang des Reaktionsgasgemischs durch das Katalysatorfestbett) gewährleistet, dies allerdings normalerweise zum Preis einer verringerten Selektivität der Zielproduktbildung.

Die Aufgabe der vorliegenden Erfindung bestand daher darin, ein verbessertes Verfahren des Langzeitbetriebes einer wie beschrieben in zwei Temperaturzonen durchgeführten heterogen katalysierten Gasphasen-Partialoxidation zur Verfügung zu stellen.

Demgemäss wurde ein Verfahren zum Langzeitbetrieb einer heterogen katalysierten partiellen Gasphasenoxidation einer organischen Ausgangsverbindung zu einer organischen Zielverbindung, bei dem man ein die organische Ausgangsverbindung und molekularen Sauerstoff enthaltendes Reaktionsgaseingangsgemisch zunächst mit der Maßgabe durch ein frisch beschicktes Katalysatorfestbett, das in zwei räumlich aufeinanderfolgenden (und in der Regel aneinandergrenzenden) Temperaturzonen A, B aufgeschüttet ist, deren Temperaturen $T^A$ und $T^B$ so beschaffen sind, dass der Betrag der Differenz $\Delta T^{BA}$ zwischen der Temperatur $T^B$ der Temperaturzone B und der Temperatur $T^A$ der Temperaturzone A und berechnet mit der höheren der beiden Temperaturen als Minuend > 0 °C beträgt, führt, dass das Reaktionsgaseingangsgemisch die Temperaturzonen A, B in der zeitlichen Abfolge "erst A" und "dann B" durchströmt, wobei sich die Temperaturzone A bis zu einem Umsatz der organischen Ausgangsverbindung von $U^A$ = 15 bis 85 mol-% erstreckt und sich in der Temperaturzone B der Umsatz der organischen Ausgangsverbindung auf einen Wert $U^B \geq$ 90 mol-% erhöht und bei dem man anschließend mit zunehmender Betriebsdauer, um der Minderung der Qualität (insbesondere um der Minderung der volumenspezifischen Aktivitäten) des Katalysatorfestbetts entgegenzuwirken, die Temperatur der Temperaturzonen A, B verändert, gefunden, das dadurch gekennzeichnet ist, dass man mit zunehmender Betriebsdauer die Temperatur derjenigen Temperaturzone, die zunächst ("anfangs") die tiefere Temperatur aufwies, erhöht (vorzugsweise im wesentlichen stetig), und die Differenz $\Delta T^{BA}$ zwischen den Temperaturen der beiden Temperaturzonen verringert (vorzugsweise im wesentlichen stetig; verringern umfasst dabei ausdrücklich auch ein zunehmendes "negativ" werden), wobei bei der Differenzbildung die Temperatur derjenigen Temperaturzone, die zunächst die höhere Temperatur aufwies, ihre Position als Minuend beibehält.

Vorstehende, und alle anderen Aussagen zum erfindungsgemäßen Verfahren in dieser Schrift, besitzen insbesondere Gültigkeit für die heterogen katalysierte Festbett-Gasphasen-Partialoxidation von Propylen zu Acrolein und/oder Acrylsäure, von iso-Buten zu Methacrolein und/oder Methacrylsäure, von (Meth)acrolein zu (Meth)acrylsäure, von Propan zu Acrolein und/oder Acrylsäure sowie von iso-Butan zu Methacrolein und/oder Methacrylsäure. Selbstredend gelten sie aber auch für alle anderen eingangs dieser Schrift benannten heterogen katalysierten partiellen Gasphasenoxidationen.

Im weiteren Verlauf dieser Schrift werden das erfindungsgemäße Verfahren und besondere Ausgestaltungen desselben insbesondere am Beispiel der heterogen katalysierten Festbett-Gasphasen-Partialoxidation von Propylen zu Acrolein, bzw. von Acrolein zu Acrylsäure erläutert und beispielhaft ausgeführt. Dies geschieht jedoch ohne Beschränkung der Allgemeingültigkeit der vorliegenden Erfindung und ist in der Regel in entsprechender Weise auf die anderen in dieser Schrift genannten heterogen katalysierten Festbett-Gasphasen-Partialoxidationen übertragbar.

Grundsätzlich kann beim erfindungsgemäßen Verfahren vorab des Ergreifens der erfindungsgemäßen Maßnahme (d.h., "anfangs") für einen vorteilhaften Langzeitbetrieb (z. B. in Abhängigkeit von der Gestaltung des frisch beschickten Katalysatorfestbetts) sowohl $T^A$ als auch $T^B$ die kleinere der beiden Temperaturen gewesen sein.

Welche der beiden Temperaturen die kleinere war, hängt nicht zuletzt auch davon ab, bei welcher Eduktbelastung des Katalysatorfestbetts das Verfahren der heterogen katalysierten Festbett-Gasphasen-Partialoxidation durchgeführt wird (selbstverständlich ist hier auch die Wahl des zu verwendenden Katalysators von Einfluss).
Bei niederen Eduktbelastungen des frischen Katalysatorfestbetts ist häufig die Bedingung $T^B - T^A < 0$ °C vorteilhaft, während mit zunehmender Eduktbelastung des Katalysatorfestbetts normalerweise die Bedingung $T^B - T^A > 0$ °C vorteilhaft ist. Grundsätzlich wird man die Differenz $T^B - T^A$ für die Durchführung des erfindungsgemäßen Verfahrens am frisch beschickten Katalysatorfestbett anwendungstechnisch vorteilhaft danach ausrichten, dass dabei für die Differenz $T^{maxA} - T^{maxB}$ ein Wert $\geq 0$ °C resultiert. Im Regelfall wird $T^{maxA} - T^{maxB}$ beim frisch beschickten Katalysatorfestbett so eingestellt, dass diese Differenz nicht mehr als 80 °C beträgt. Anwendungstechnisch zweckmäßig beträgt $T^{maxA} - T^{maxB}$ für das frisch beschickte Katalysatorfestbett $\geq 3$ °C und $\leq 70$ °C, besonders vorteilhaft $\geq 5$ und $\leq 60$ °C, bzw. $\leq 50$ °C. Ganz besonders vorteilhaft beträgt diese Differenz $\geq 5$ und $\leq 40$ °C, bzw. $\geq 5$ und 25 °C, oder $\geq 5$ und $\leq 20$ °C bzw. $\leq$ 15 °C. Häufig beträgt diese Differenz auch $\geq 0$ bis $\leq 5$ °C.

Gemäß der erfindungsgemäßen Verfahrensweise ist es nun erforderlich, im Langzeitbetrieb des erfindungsgemäßen Verfahrens an ein und demselben Katalysatorfestbett, die Temperatur derjenigen Temperaturzone, die zunächst die tiefere Temperatur aufwies, zu erhöhen, und die Differenz $\Delta T^{BA}$ zwischen den Temperaturen der beiden Temperaturzonen zu verringern, wobei bei der Differenzbildung die Temperatur derjenigen Temperaturzone, die zunächst die höhere Temperatur aufwies, ihre Position als Minuend beibehält.

Unter der Voraussetzung, dass zunächst (zuvor, "anfangs") die Temperatur der Temperaturzone B ($T^B$) die höhere Temperatur war, würde man gemäß der in dieser Schrift gegebenen Lehre im Langzeitbetrieb die Temperatur der Temperaturzone A ($T^A$) erhöhen. Eine damit einhergehende Verringerung der Differenz $\Delta T^{BA}$ kann nun grundsätzlich auf drei verschiedene Betriebsweisen erreicht werden:

a) die Temperatur der Temperaturzone B wird ebenfalls erhöht, aber nicht so stark wie die Temperatur der Temperaturzone A;
b) die Temperatur der Temperaturzone B wird beibehalten;
c) die Temperatur der Temperaturzone B wird erniedrigt.

Unter der Voraussetzung, dass zunächst (zuvor) die Temperatur der Temperaturzone A ($T^A$) die höhere Temperatur war, würde man gemäß der in dieser Schrift gegebenen Lehre im Langzeitbetrieb die Temperatur der Temperaturzone B ($T^B$) erhöhen. Eine damit einhergehende Verringerung der Differenz $\Delta T^{BA}$ kann nun grundsätzlich auf die drei nachfolgenden Betriebsweisen erreicht werden:

a) die Temperatur der Temperaturzone A wird ebenfalls erhöht, aber nicht so stark wie die Temperatur der Temperaturzone B;
b) die Temperatur der Temperaturzone A wird beibehalten;
c) die Temperatur der Temperaturzone A wird erniedrigt.

Insbesondere im Fall der vorgenannten Betriebsweisen b) und c), grundsätzlich aber auch bei der Betriebsweise a), besteht die Möglichkeit, dass beim erfindungsgemäßen Verfahren $\Delta T^{BA}$ im Verlauf des Langzeitbetriebes (verglichen mit dem Wert derselben Größe bei frischem Katalysatorfestbett) sein Vorzeichen wechselt. Ganz allgemein wird der Betrag von $\Delta T^{BA}$ in der Regel 60 °C, meist 50 °C jedoch nicht überschreiten. D.h., der Betrag $\Delta T^{BA}$ kann beim erfindungsgemäßen Verfahren z. B. $\geq 0$ bis 60 °C, oder $\geq 1$ bis 55 °C, oder $\geq 5$ bis 50 °C, oder $\geq 10$ bis 40 °C, oder $\geq 15$ bis 35 °C, oder $\geq 20$ bis 30 °C betragen.

Erfindungsgemäß vorteilhaft wird in der Regel so verfahren, dass $\Delta T^{BA}$ im Langzeitbetrieb (z. B. innerhalb einer Betriebsdauer von 2 Monaten, oder von 6 Monaten, oder von 12 Monaten, oder von 18 Monaten, oder von 24 Monaten, oder von 30 Monaten, oder von 36 Monaten oder mehr) sein Vorzeichen möglichst lange nicht wechselt.

An dieser Stelle sei festgehalten, dass die Temperaturen der Temperaturzonen A und B ($T^A$ und $T^B$) im großtechnischen Betrieb aus unterschiedlichen Gründen gewissen Schwankungen (in der Regel innerhalb des Intervalls $\pm$ 20 °C bzw. $\pm$ 10°C liegend) unterliegen können (beispielsweise dann, wenn eine Zwischenregenerierung gemäß der DE-A 10351269 vorgenommen wird; unmittelbar nach erfolgter Zwischenregenerierung (im Vergleich zum Betrieb unmittelbar vor der Zwischenregenerierung) sind in der Regel geringere Temperaturen (in Einzelfällen kann dieser Temperaturunterschied auch bis zu 40 °C oder mehr betragen) der Temperaturzonen ausreichend, um unter ansonsten unveränderten Bedingungen den selben, auf den Einmaldurchgang des Reaktionsgasgemischs durch das Katalysatorfestbett bezogenen, Eduktumsatz zu gewährleisten). In diesem Fall trägt man den tatsächlichen Verlauf der Temperatur der jeweiligen Zone über die Zeit auf und legt durch die Messpunkte nach der von Legendre und Gauß entwickelten Methode der kleinsten Summe der Abweichungsquadrate eine Ausgleichskurve. Sind auf der Grundlage dieser Ausgleichskurven die erfindungsgemäßen Merkmale erfüllt, wird von der erfindungsgemäßen Verfahrensweise Gebrauch gemacht.

Für den Fall, dass im Verlauf einer erfindungsgemäßen heterogen katalysierten partiellen Gasphasen-Partialoxidation aufgrund von z. B. veränderter Marktnachfrage oder anderweitig veränderten Rahmenbedingungen im Rahmen des Langzeitbetriebs ein und desselben Katalysatorfestbetts Rahmenbedingungen des Verfahrens wie z. B. die Belastung des Katalysatorfestbetts, oder die Belastung des Katalysatorfestbetts und der auf den Einmaldurchgang des Reaktionsgasgemischs durch das Katalysatorfestbett bezogene Eduktumsatz (Umsatz an der organischen Ausgangsverbindung) mit unmittelbarer Rückwirkung (eine solche Veränderung wäre auch eine Erhöhung des Arbeitsdrucks gemäß der DE-A 10 2004 025 445) auf die Temperatur der Temperaturzonen A, B verändert werden, um sie nachfolgend im weiteren Betrieb so verändert über einen längeren Zeitabschnitt (Betriebsabschnitt) im wesentlichen beizubehalten, so liegt eine erfindungsgemäße Verfahrensweise auch dann vor, wenn in diesem nachfolgenden längeren Betriebsabschnitt mit Bezug auf das Katalysatorfestbett und dessen Betrieb (im wesentlichen "unmittelbar") nach der vorgenannten Veränderung als Betrieb eines "frischen Katalysatorfestbetts" die erfindungsgemäßen kennzeichnenden Merkmale erfüllt werden.

Ferner soll unter dem Verfahren einer heterogen katalysierten partiellen Gasphasenoxidation einer organischen Ausgangsverbindung am frisch beschickten Katalysatorfestbett die Ausübung des Verfahrens nach Abschluss einer gegebenenfalls auftretenden Formierung des Katalysatorfestbetts, d.h., nach Erreichen des quasi-stationären Betriebszustandes verstanden werden.

Ganz generell kann man beim erfindungsgemäßen Verfahren die erfindungsgemäß vorzunehmenden Veränderungen von $T^A$, $T^B$ und von $\Delta T^{BA}$ so vornehmen, dass auch im Verlauf des Langzeitbetriebs des Katalysatorfestbetts eine Differenz $T^{maA}$ - $T^{maxB} \geq 0$ °C weitgehend erhalten bleibt (z. B. fortwährend im Bereich $\geq 0$ °C und $\leq 80$ °C, oder $\geq 1$ °C und $\leq 70$ °C, oder $\geq 2$ °C und $\leq 60$ °C, oder $\geq 3$ °C und $\leq 50$ °C, oder $\geq 4$ °C und $\leq 40$ °C, oder $\geq 5$ °C und $\leq 30$ °C, oder $\geq 5$ °C und $\leq 25$ °C, oder $\geq 5$ °C und $\leq 20$ °C bzw. $\leq 15$ °C liegend; oder auch fortwährend im Bereich $\geq 0$ °C und $\leq 5$ °C liegend).

Eine solche Ausführungsform der erfindungsgemäßen Verfahrensweise ist eine bevorzugte, da sie besonders hohe Zielproduktselektivitäten bedingt. Im Regelfall wird sie bei Anwendung der vorstehend ausgeführten Betriebsweisen c) erreicht.

Die erfindungsgemäß vorzunehmenden Veränderungen von $T^A$, $T^B$ und von $\Delta T^{BA}$ können aber auch so vorgenommen werden, dass im Verlauf des Langzeitbetriebs des Katalysatorfestbetts die Differenz $T^{maxA}$ - $T^{maxB}$ von $\geq$ 0°C nach < 0°C wechselt (z.B. von $\leq$ 80°C nach bis zu -20°C, oder bis zu -10°C, oder bis zu -5°C, oder von $\leq$ 60°C nach bis zu -20°C, oder bis zu -10°C, oder bis zu -5°C, oder von $\leq$ 40°C, bzw. von $\leq$ 20°C nach bis zu -20°C, oder bis zu -10°C, oder bis zu -5°C, oder von $\leq$ 10°C auf bis zu -20°C, oder bis zu -10°C, oder bis zu -5°C).

Eine solche Ausführungsform der erfindungsgemäßen Verfahrensweise ist ebenfalls eine bevorzugte, da sie besonders lange Standzeiten (Gesamtbetriebszeiten) des Katalysatorfestbetts ermöglicht. Spätestens beim Erreichen von $T^{maxA}$ - $T^{maxB}$ = -20°C wird man jedoch das Katalysatorfestbett normalerweise vollständig oder wenigstens teilweise gegen ein frisches Katalysatorfestbett austauschen. Im Regelfall wird eine solche Ausführungsform bei Anwendung der vorstehend ausgeführten Betriebsweisen a), weniger ausgeprägt aber auch bei den Betriebsweisen b) erreicht.

Selbstredend kann man beim erfindungsgemäßen Verfahren aber auch erfindungsgemäß vorteilhaft zunächst gemäß einer Betriebsweise c) verfahren (die zunächst (zuvor) tiefere der beiden Temperaturen $T^A$, $T^B$ wird (vorzugsweise im wesentlichen stetig) erhöht und die zuvor (zunächst) höhere der beiden Temperaturen $T^A$, $T^B$ wird (vorzugsweise im wesentlichen stetig) erniedrigt) und das erfindungsgemäße Verfahren im Langzeitbetrieb so zunächst unter dem Gesichtspunkt einer maximalen Selektivität der Zielproduktbildung (bei ansonsten im wesentlichen unveränderten Verfahrensbedingungen wie die Zusammensetzung des Reaktionsgaseingangsgemischs, die Belastung des Katalysatorfestbetts mit organischer Ausgangsverbindung und Reaktionsgasgemisch und auf den Einmaldurchgang des Reaktionsgasgemischs durch das Katalysatorfestbett bezogenem Eduktumsatz) betreiben.

Anschließend kann dann zum Zweck der Maximierung der Standzeit des Katalysatorfestbetts zu einer Betriebsweise b) oder a) gewechselt werden.

Grundsätzlich kann beim erfindungsgemäßen Verfahren beliebig zwischen den Betriebsweisen a), b) und c) hin- und hergesprungen werden, so lange die erfindungsgemäß zu erfüllenden Merkmale erfüllt werden.

Generell macht auch derjenige vom erfindungsgemäßen Verfahren Gebrauch, der das erfindungsgemäße Verfahren nur über einen bestimmten Zeitbereich des Langzeitbetriebs betreibt und vorab des teilweisen oder vollständigen Ersatzes des Katalysatorfestbetts durch ein Frischbett die erfindungsgemäße Langzeitbetriebsweise verlässt.

In der Regel wird man spätestens dann vom erfindungsgemäßen Verfahren Gebrauch machen, wenn sich das Katalysatorfestbett in einem solchen Zustand befindet, dass $U^B$ unter ansonsten unveränderten Verfahrensbedingungen um wenigstens 0,2, bzw. wenigstens 0,3, bzw. wenigstens 0,4, oder wenigstens 0,5 Molprozentpunkte niederer läge als der Wert für $U^B$ unter denselben Verfahrensbedingungen am frischen Katalysatorbett.

Ganz generell wird im Langzeitbetrieb des erfindungsgemäßen Verfahrens in der Regel $U^A$ = 15 bis 85 mol-% und $U^B \geq$ 90 mol-% beibehalten. Insbesondere im Fall einer heterogen katalysierten Partialoxidation von Propylen zu Acrolein bzw. von Acrolein zu Acrylsäure (in der Regel aber auch bei den anderen möglichen heterogen katalysierten Gasphasen-Partialoxidationen) ist es bevorzugt, wenn $U^B \geq$ 92 mol-%, oder $\geq$ 94 mol-%, oder $\geq$ 96 mol-% beträgt. Im Fall der heterogen katalysierten Partialoxidation von Acrolein zu Acrylsäure beträgt $U^B$ während des Langzeitbetriebs sogar besonders vorteilhaft durchgehend $\geq$ 98 mol-%, oder $\geq$ 99 mol-%, vielfach sogar $\geq$ 99,5 oder $\geq$ 99,9 mol-%.

Die zu verwendenden Katalysatoren und sonstigen Verfahrensbedingungen wird man im übrigen anwendungstechnisch zweckmäßig so wählen, dass die Selektivität der Zielproduktbildung, bezogen auf den Einmaldurchgang des Reaktionsgasgemischs durch das Katalysatorfestbett, $\geq$ 80 mol-%, oder $\geq$ 90 mol-%, vielfach sogar $\geq$ 92 mol-%, oder $\geq$ 94 mol-%, oder $\geq$ 96 mol-% beträgt.

Anwendungstechnisch zweckmäßig erfolgt die Durchführung des erfindungsgemäßen Verfahrens bevorzugt in den bereits angesprochenen Zweizonen-Vielkontaktrohr-Reaktoren. Der radiale Temperaturgradient des Wärmeträgers innerhalb einer Temperaturzone beträgt dabei in der Regel 0,01 bis 5°C, häufig 0,1 bis 2°C und ist erfindungsgemäß vorteilhaft möglichst gering.

In typischer Weise wird die Temperatur des Wärmeträgers vom Eintritt in die Temperaturzone bis zum Austritt aus der Temperaturzone (bedingt duch die Exothermie der Reaktion) um 0 bis 15°C ansteigen. In typischer Weise wird das vorgenannte $\Delta T$ erfindungsgemäß 1 bis 10°C, oder 2 bis 8°C, oder 3 bis 6°C betragen.

Grundsätzlich kann sie jedoch auch in sonstigen zwei Temperaturzonen aufweisenden Reaktoren vom Typ eines indirekten Wärmeaustauschers durchgeführt werden.

In der Regel wird sich der Langzeitbetrieb des erfindungsgemäßen Verfahrens auf wenigstens 2 Betriebsmonate, oder auf wenigstens 4 Betriebsmonate, oder wenigstens 6 Betriebsmonate, bzw. wenigstens 1 Betriebsjahr, oder wenigstens 2 Betriebsjahre und teilweise sogar auf bis zu 10 Betriebsjahre oder mehr erstrecken. Erreichen die Werte für $T^{maxA}$, $T^{maxB}$ die Temperaturen, bei denen die thermische Behandlung der Katalysatorvorläufermasse zur Herstellung des Katalysators erfolgte, wird man das Katalysatorfestbett anwendungstechnisch zweckmäßig wenigstens teilweise oder vollständig durch ein frisches Katalysatorfestbett ersetzen.

Die Vorteilhaftigkeit der erfindungsgemäßen Verfahrensweise mit Blick auf die Selektivität der Zielproduktbildung liegt vermutlich darin begründet, dass sie einer Verfahrensweise entgegenwirkt, bei der in der Temperaturzone A bereits gebildetes Zielprodukt innerhalb der Temperaturzone B zu hohe Reaktionstemperaturen durchlaufen muss, die eine Vollverbrennung des gebildeten Zielprodukts fördern. Gleichzeitig eröffnet die erfindungsgemäße Verfahrensweise die Möglichkeit, über den Langzeitbetrieb betrachtet, darauf hinzuwirken, dass das katalytische Potential des verfügbaren Katalysatorfestbetts im wesentlichen über das gesamte Katalysatorfestbett vollständig abgerufen wird. Dabei macht sich die vorliegende Erfindung den Sachverhalt zu eigen, dass die Deaktivierung des Katalysatorfestbetts bei Durchführung des erfindungsgemäßen Verfahrens am frisch beschickten Katalysatorfestbett über das Katalysatorfestbett nicht homogen erfolgt. Vielmehr wird sie u.a. dort besonders ausgeprägt erfolgen, wo sich die $T^{maxA}$ bzw. $T^{maxB}$ befinden, weshalb sich deren Position längs des Katalysatorfestbetts im Langzeitbetrieb ohne erfindungsgemäße Änderung der Temperatur der Temperaturzonen A, B normalerweise in natürlicher Weise in Strömungsrichtung des Reaktionsgasgemischs verschieben würde.

Erfindungsgemäß bevorzugt wird man die erfindungsgemäße Verfahrensweise kombiniert mit einer Zwischenregenerierung gemäß der Lehre der DE-A 103 51 269 anwenden. Ferner wird man normalerweise, bevor das Katalysatorfestbett vollständig ausgetauscht wird, einen Teilbettwechsel gemäß der Lehre der DE-A 10232748 bzw. der WO 2004/009525 vornehmen. Dabei kann sich der Teilkatalysatorfestbettwechsel in allen Fällen in Strömungsrichtung des Reaktionsgasgemischs auf bis zu 80 %, oder nur auf bis zu 70 %, oder nur auf bis zu 60 %, oder nur auf bis zu 50 %, oder nur auf bis zu 40 %, oder nur auf bis zu 30 %, oder bevorzugt auf bis zu 25 %, besonders bevorzugt auf 30 bis 50 % und ganz besonders bevorzugt auf 35 bis 45 % der Schüttlänge des jeweiligen Katalysatorfestbetts erstrecken (eine zu 100 % aus Inertmaterial bestehende Deckbeschickung (die Erstbeschickung aus der Strömungssicht) wird dabei nicht zum Katalysatorfestbett zugehörig betrachtet. In entsprechender Weise wird für die Belange der vorliegenden Erfindung normalerweise eine zu 100 % aus Inertmaterial bestehende Abschlussbeschickung (die Endbeschickung aus der Strömungssicht) nicht zum Katalysatorfestbett zugehörig betrachtet. Eine zu 100 % aus Inertmaterial bestehende Zwischenbeschickung wird üblicherweise jedoch zum Katalysatorfestbett zugehörig betrachtet. Zweckmäßigerweise beträgt der vorgenannte Prozentsatz für einen Teilkatalysatorwechsel häufig nicht weniger als 5, bzw. nicht weniger als 10 bzw. nicht weniger als 20 %.

Handelt es sich beim erfindungsgemäßen Verfahren am frisch beschickten Katalysatorfestbett um eine heterogen katalysierte Festbett-Gasphasen-Partialoxidation von Propylen zu Acrolein, stellen sich die als vorteilhaft empfohlenen Differenzen $T^{maxA}$ - $T^{maxB}$ (z.B. $\geq$ 0°C und $\leq$ 80°C, häufig $\geq$ 1°C und $\leq$ 70°C, oft $\geq$ 2°C und $\leq$ 60°C, vielfach $\geq$ 3°C und $\leq$ 50°C, vorteilhaft $\geq$ 4°C und $\leq$ 40°C, bevorzugt $\geq$ 5°C und $\leq$ 30°C, oder $\leq$ 20°C, besonders bevorzugt $\geq$ 5°C

und ≤ 15°C, oder auch ≥ 0°C und ≤ 5°C) im Fall von eher niederen (≥80 NI/I•h und ≤ 130 NI/I•h bzw. ≤ 110 NI/I•h) Propenbelastungen des frischen Katalysatorfestbetts häufig dann ein, wenn einerseits sowohl die Temperatur der Temperaturzone A als auch die Temperatur der Temperaturzone B im Bereich von 290 bis 380°C liegt und andererseits die Differenz zwischen der Temperatur der Temperaturzone B ($T^B$) und der Temperatur der Temperaturzone A ($T^A$), d. h., $\Delta T^{BA} = T^A - T^B > 0°C$ und ≤ 20°C bzw. ≤ 10°C, oder > 0°C und ≤ 5°C, oder häufig > 0°C und ≤ 3°C beträgt (in diesem Fall würde man im erfindungsgemäßen Langzeitbetrieb in erfindungsgemäß notwendiger Weise die Temperatur der Temperaturzone B erhöhen (vorzugsweise stetig) und auf die Temperatur der Temperaturzone A wenigstens eine der Betriebsweisen a) bis c) anwenden (das ganze vorzugsweise so, dass $T^{maxA} - T^{maxB} \geq 0°C$ erhalten bleibt). Vorzugsweise verbleiben die Temperaturen der beiden Temperaturzonen A, B dabei im Temperaturbereich von 290 bis 380°C.

Bei Ausübung der heterogen katalysierten Festbett-Gasphasen-Partialoxidation von Propylen zu Acrolein unter erhöhten Propylenbelastungen des frischen Katalysatorfestbetts (> 130NI/I•h, oder ≥ 140NI/I•h, bzw. ≥ 160NI/I•h und in der Regel ≤ 200 bzw. ≤ 300NI/I•h, bzw. normalerweise ≤ 600NI/I•h) stellen sich die als vorteilhaft empfohlenen Differenzen $T^{maxA} - T^{maxB}$ (siehe vorstehend) am frisch beschickten Katalysatorfestbett normalerweise dann ein, wenn einerseits sowohl die Temperatur der Temperaturzone A als auch die Temperatur der Temperaturzone B im Bereich von 290 bis 380 °C liegt und andererseits die Differenz zwischen der Temperatur der Temperaturzone B ($T^B$) und der Temperatur der Temperaturzone A ($T^A$), d.h., $\Delta T^{BA} = T^B - T^A \geq 0°C$ und ≤ 50 °C, oder ≥ 5 °C und ≤ 45 °C, oder ≥ 10 °C und ≤ 40 °C, oder ≥ 15 °C und ≤ 30 °C, oder ≤ 35 °C (z.B. 20 °C oder 25 °C) beträgt (in diesem Fall würde man im erfindungsgemäßen Langzeitbetrieb in erfindungsgemäß notwendiger Weise die Temperatur der Temperaturzone A erhöhen (vorzugsweise stetig) und auf die Temperatur der Temperaturzone B wenigstens eine der Betriebsweisen a) bis c) anwenden (das ganze vorzugsweise so, dass $T^{maxA} - T^{maxB} \geq 0°C$ erhalten bleibt; vorzugsweise die Betriebsweise c)). Vorzugsweise verbleiben die Temperaturen der beiden Temperaturzonen A, B dabei im Temperaturbereich von 290 bis 380 °C.

Mit Vorteil liegt die Temperatur der Temperaturzone A bei einer erfindungsgemäßen heterogen katalysierten Festbett-Gasphasen-Partialoxidation von Propylen zu Acrolein (unabhängig von der Propylenbelastung des Katalysatorfestbetts) während des erfindungsgemäßen Langzeitbetriebs (und während des Betriebs der Frischbeschickung) im bevorzugten Bereich von 305 bis 365 °C bzw. im besonders bevorzugten Bereich von 310 bis 340 °C.

Die Propylenbelastung des Katalysatorfestbetts kann somit bei einer erfindungsgemäßen heterogen katalysierten Festbett-Gasphasen-Partialoxidation von Propylen zu Acrolein z.B. ≥ 80 NI/I·h oder ≥ 90 NI/I·h und ≤ 300 NI/I·h, oder ≤ 600 NI/I·h, oder ≥110 NI/I·h und ≤ 280 NI/I·h, oder ≥ 130 NI/I·h und ≤ 260 NI/I·h, oder ≥ 150 NI/I·h und ≤ 240 NI/ I•h, oder ≥ 170 NI/I•h und ≤ 220 NI/I•h, oder ≥ 190 NI/I•h und ≤ 200 NI/I•h betragen. D. h., als erfindungsgemäße Propylenbelastung des Katalysatorfestbetts kommt auch der Bereich ≥ 120 NI/I•h und ≤ 200 NI/I•h bzw. ≤ 300 NI/I•h in Betracht.

Erfindungsgemäß bevorzugt erstreckt sich die Temperaturzone A beim Betrieb des frisch beschickten Katalysatorfestbetts bis zu einem Umsatz $U^A$ des Propylens von 30 bzw. von 40 bis 80 mol-%, oder von 50 bis 70 mol-%, bzw. von 60 bis 70 mol-%. Erfindungsgemäß vorteilhaft liegen auch während des erfindungsgemäßen Langzeitbetriebs einer heterogen katalysierten Festbett-Gasphasen-Partialoxidation von Propylen zu Acrolein die Propylenumsätze $U^A$ in einem der vorgenannten Umsetzbereiche. Tendenziell verschieben sie sich während des Langzeitbetriebs zu geringeren molaren Prozentsätzen.

Handelt es sich beim erfindungsgemäßen Verfahren am frisch beschickten Katalysatorfestbett um eine heterogen katalysierte Festbett-Gasphasen-Partialoxidation von Acrolein zu Acrylsäure, stellen sich die als vorteilhaft empfohlenen Differenzen $T^{maxA} - T^{maxB}$ (z.B. ≥ 0 °C und ≤ 80 °C, häufig ≥ 1 °C und ≤ 70 °C, oft ≥ 2 °C und ≤ 60 °C, vielfach ≥ 3 °C und ≤ 50 °C, vorteilhaft ≥ 4 °C und ≤ 40 °C, bevorzugt ≥ 5 °C und ≤ 30 °C, oder ≤ 20 °C, besonders bevorzugt ≥ 5 °C und ≤ 15 °C, oder auch ≥ 0 °C und ≤ 5 °C) im Fall von eher niederen (≥ 60 NI/I•h bzw. ≥ 70 NI/I•h und ≤ 120 NI/ I•h, bzw. ≤ 100 NI/I•h) Acroleinbelastungen des frischen Katalysatorfestbetts häufig dann ein, wenn einerseits sowohl die Temperatur der Temperaturzone A als auch die Temperatur der Temperaturzone B im Bereich von 230 bis 320°C liegt und andererseits die Differenz zwischen der Temperatur der Temperaturzone B ($T^B$) und der Temperatur der Temperaturzone A ($T^A$), d.h., $\Delta T^{BA} = T^A - T^B > 0$ °C und ≤ 20 °C bzw. ≤ 10 °C, oder > 0 °C und ≤ 5 °C, oder häufig > 0 °C und ≤ 3 °C beträgt (in diesem Fall würde man im erfindungsgemäßen Langzeitbetrieb in erfindungsgemäß notwendiger Weise die Temperatur der Temperaturzone B erhöhen (vorzugsweise stetig) und auf die Temperatur der Temperaturzone A wenigstens eine der Betriebsweisen a) bis c) anwenden (das ganze vorzugsweise so, dass $T^{maxA} - T^{maxB} \geq 0°C$ erhalten bleibt). Vorzugsweise verbleiben die Temperaturen der beiden Temperaturzonen A, B dabei im Temperaturbereich von 230 bis 320 bzw. bis 340 °C.

Bei Ausübung der heterogen katalysierten Festbett-Gasphasen-Partialoxidation von Acrolein zu Acrylsäure unter erhöhten Acroleinbelastungen des frischen Katalysatorfestbetts (> 120 NI/I•h (gegebenenfalls aber auch schon bei > 100 NI/I•h), bzw. ≥ 130 NI/I•h, oder ≥ 140 NI/I•h, oder ≥ 150 NI/I•h und in der Regel ≤ 175, bzw. ≤ 200, bzw. ≤ 300 NI/I•h, bzw. normalerweise ≤ 600 NI/I•h) stellen sich die als vorteilhaft empfohlenen Differenzen $T^{maxA} - T^{maxB}$ (siehe vorstehend) am frischen Katalysatorfestbett normalerweise dann ein, wenn einerseits sowohl die Temperatur der Temperaturzone A als auch die Temperatur der Temperaturzone B im Bereich von 230 bis 320 bzw. bis 340 °C liegt und

anderseits die Differenz zwischen der Temperatur der Temperaturzone B ($T^B$) und der Temperatur der Temperaturzone A ($T^A$), d.h., $\Delta T^{BA} = T^B - T^A \geq 0$ °C und $\leq 50$ °C, oder $\geq 5$ °C und $\leq 45$ °C, oder $\geq 10$ °C und $\leq 40$ °C, oder $\geq 15$ °C und $\leq 30$ °C oder $\leq 35$ °C (z.B. 20 °C oder 25 °C), oder $\geq 10$ °C und $\leq 25$ °C, bzw. $\leq 20$ °C, oder $\leq 15$ °C beträgt (in diesem Fall würde man im erfindungsgemäßen Langzeitbetrieb in erfindungsgemäß notwendiger Weise die Temperatur der Temperaturzone A erhöhen (vorzugsweise stetig) und auf die Temperatur der Temperaturzone B wenigstens eine der Betriebsweisen a) bis c) anwenden (das ganze vorzugsweise so, dass $T^{maxA} - T^{maxB} \geq 0$ °C erhalten bleibt; vorzugsweise die Betriebsweise c)).

Vorzugsweise verbleiben die Temperaturen der beiden Temperaturzonen A, B dabei im Temperaturbereich von 230 bis 320 bzw. bis 340 °C.

Mit Vorteil liegt die Temperatur der Temperaturzone A bei einer erfindungsgemäßen heterogen katalysierten Festbett-Gasphasen-Partialoxidation von Acrolein zu Acrylsäure (unabhängig von der Acroleinbelastung des Katalysatorfestbetts) während des erfindungsgemäßen Langzeitbetriebs (und während des Betriebs der Frischbeschickung) im bevorzugten Bereich von 250 bis 300 °C bzw. im besonders bevorzugten Bereich von 260 bis 280 °C.

Die Acroleinbelastung des Katalysatorfestbetts kann somit bei einer erfindungsgemäßen heterogen katalysierten Festbett-Gasphasen-Partialoxidation z.B. $\geq 60$ Nl/l•h, oder $\geq 70$ Nl/l•h, bzw. $\geq 90$ Nl/l•h und in der Regel $\leq 300$ Nl/l•h bzw. üblicherweise $\leq 600$ Nl/l•h, oder $\geq 110$ Nl/l•h und $\leq 280$ Nl/l•h, oder $\geq 130$ Nl/l•h und $\leq 260$ Nl/l•h, oder $\geq 150$ Nl/l•h und $\leq 240$ Nl/l•h, oder $\geq 170$ Nl/l•h und $\leq 220$ Nl/l•h, oder $\geq 190$ Nl/l•h und $\leq 200$ Nl/l•h betragen.

D. h., die Acroleinbelastung des Katalysatorfestbetts kann erfindungsgemäß auch $\geq 90$ bis $\leq 150$ Nl/l•h, bzw. bis $\leq 300$ Nl/l•h betragen.

Erfindungsgemäß bevorzugt erstreckt sich die Temperaturzone A beim Betrieb des frisch beschickten Katalysatorfestbetts bis zu einem Umsatz $U^A$ des Acroleins von 30 bzw. von 40 bis 85 mol-%, oder von 50 bis 85 mol-%, bzw. von 60 bis 85 mol-%.

Erfindungsgemäß vorteilhaft liegen auch während des erfindungsgemäßen Langzeitbetriebs einer heterogen katalysierten Festbett-Gasphasen-Partialoxidation von Acrolein zu Acrylsäure die Acroleinumsätze $U^A$ in einem der vorgenannten Umsatzbereiche. Tendenziell verschieben sie sich während des Langzeitbetriebs zu geringeren molaren Prozentsätzen.

Der Arbeitsdruck kann sowohl bei der erfindungsgemäßen heterogen katalysierten Gasphasen-Partialoxidation von Propylen zu Acrolein, als auch von Acrolein zu Acrylsäure unterhalb von Normaldruck (z.B. 0,5 bis zu 1 atm) oder oberhalb von Normaldruck liegen. Typischerweise wird der Arbeitsdruck bei beiden erfindungsgemäßen Partialoxidationen bei Werten von 1 bis 5 atm, häufig 1 bis 3 atm liegen. Normalerweise wird der Arbeitsdruck (Reaktionsdruck) bei beiden Partialoxidationen 100 atm nicht überschreiten.

In der Regel wird der Propylenumsatz $U^B$ bei einer erfindungsgemäßen heterogen katalysierten partiellen Gasphasenoxidation von Propylen zu Acrolein (bezogen auf den Einmaldurchgang des Reaktionsgasgemischs durch das Katalysatorfestbett) $\geq 90$ mol-%, oder $\geq 92$ mol-%, oder $\geq 94$ mol-% betragen. Die Selektivität der Wertproduktbildung (Summe aus Acroleinbildung und Acrylsäurenebenproduktbildung) wird bei in an sich bekannter Weise geeigneter Wahl der Festbettkatalysatorschüttung regelmäßig $\geq 80$ mol-%, oder $\geq 85$ mol-%, oder $\geq 90$ mol-%, oder $\geq 92$ mol-%, oder $\geq 94$ mol-%, häufig $\geq 95$ mol-%, oder $\geq 96$ mol-% bzw. $\geq 97$ mol-% betragen.

In der Regel wird der Acroleinumsatz $U^B$ bei einer erfindungsgemäßen heterogen katalysierten partiellen Gasphasenoxidation von Acrolein zu Acrylsäure (bezogen auf den Einmaldurchgang des Reaktionsgasgemischs durch das Katalysatorfestbett) $\geq 90$ mol-%, oder $\geq 92$ mol-%, oder $\geq 94$ mol-%, oder $\geq 96$ mol-%, oder $\geq 98$ mol-% und häufig sogar $\geq 99$ mol-% und mehr betragen. Die Selektivität der Acrylsäurebildung wird bei in an sich bekannter Weise geeigneter Wahl der Festbettkatalysatorschüttung regelmäßig $\geq 80$ mol-%, oder $\geq 85$ mol-%, oder $\geq 90$ mol-%, oder $\geq 92$ mol-%, oder $\geq 94$ mol-%, häufig $\geq 95$ mol-%, oder $\geq 96$ mol-% bzw. $\geq 97$ mol-% betragen.

Das molare Verhältnis von $O_2 : C_3H_6$ im Reaktionsgaseingangsgemisch für eine erfindungsgemäße Partialoxidation von Propylen zu Acrolein wird erfindungsgemäß normalerweise $\geq 1$ betragen. Üblicherweise wird dieses Verhältnis bei Werten $\leq 3$ liegen. Häufig beträgt das molare Verhältnis von $O_2 : C_3H_6$ für vorgenannte Reaktion erfindungsgemäß $\geq 1,2$ bzw. $\geq 1,5$ und $\leq 2,0$.

Das molare Verhältnis von $O_2$:Acrolein im Reaktionsgaseingangsgemisch für eine erfindungsgemäße Partialoxidation von Acrolein zu Acrylsäure wird erfindungsgemäß normalerweise $\geq 0,5$ betragen. Üblicherweise wird auch dieses Verhältnis bei Werten $\leq 3$ liegen. Häufig beträgt das molare Verhältnis von $O_2$:Acrolein für vorgenannte Reaktion erfindungsgemäß $\geq 1,5$ und $\leq 2,0$.

Sowohl für eine erfindungsgemäße heterogen katalysierte Partialoxidation von Propylen zu Acrolein, als auch von Acrolein zu Acrylsäure ist es günstig, wenn das Produktgasgemisch noch (z. B. bis zu 3 Vol.-%) nicht umgesetzten molekularen Sauerstoff enthält.

Als (frische) Katalysatoren für das Katalysatorfestbett (die Festbettkatalysatorschüttung) einer erfindungsgemäßen Gasphasen-Partialoxidation von Propylen zu Acrolein kommen alle diejenigen in Betracht, deren Aktivmasse wenigstens ein Molybdän und/oder Wolfram sowie wenigstens eines der Elemente Wismut, Tellur, Antimon, Zinn und Kupfer enthaltendes Multimetalloxid ist. Dazu gehören insbesondere solche Katalysatoren, deren Aktivmasse wenigstens ein Mo, Bi und Fe enthaltendes Multimetalloxid ist.

Dies sind insbesondere die Multimetalloxidaktivmassen der allgemeinen Formel I der DE-A 199 55 176, die Multimetalloxidaktivmassen der allgemeinen Formel I der DE-A 199 48 523, die Multimetalloxidaktivmassen der allgemeinen Formel I der DE-A 199 48 523, die Multimetalloxidaktivmassen der allgemeinen Formeln I, II und III der DE-A 101 01 695, die Multimetalloxidaktivmassen der allgemeinen Formeln I, II und III der DE-A 199 48 248 und die Multimetalloxidaktivmassen der allgemeinen Formeln I, II und III der DE-A 199 55 168 sowie die in der EP-A 700 714 genannten Multimetalloxidaktivmassen.

Ferner eignen sich für die frische Festbettkatalysatorschüttung einer solchen Propylenpartialoxidation die Mo, Bi und Fe enthaltenden Multimetalloxidkatalysatoren die in den Schriften Research Disclosure Nr. 497012 vom 29.08.2005, DE-A 100 46 957, DE-A 100 63 162, DE-C 33 38 380, DE-A 199 02 562, EP-A 15 565, DE-C 23 80 765, EP-A 807 465, EP-A 279 374, DE-A 33 00 044, EP-A 575 897, US-A 4,438,217, DE-A 198 55 913, WO 98/24746, DE-A 197 46 210 (diejenigen der allgemeinen Formel II), JP-A 91/294239, EP-A 293 224 und EP-A 700 714 offenbart werden. Dies gilt insbesondere für die beispielhaften (einschließlich der Vergleichsbeispiele) Ausführungsformen in diesen Schriften, unter denen jene der Research Disclosure Nr. 497012, der EP-A 15 565, der EP-A 575 897, der DE-A 197 46 210 und der DE-A 198 55 913 besonders bevorzugt werden. Besonders hervorzuheben sind in diesem Zusammenhang ein Katalysator gemäß Beispiel 1c aus der EP-A 15 565 sowie ein in entsprechender Weise herzustellender Katalysator, dessen Aktivmasse jedoch die Zusammensetzung $Mo_{12}Ni_{6,5}Zn_2Fe_2Bi_1 P_{0,0065}K_{0,06}Ox \cdot 10 SiO_2$ aufweist. Ferner sind hervorzuheben das Beispiel mit der laufenden Nr. 3 aus der DE-A 198 55 913 (Stöchiometrie: $Mo_{12}Co_7Fe_3Bi_{0,6}K_{0,08}Si_{1,6}Ox$) als Hohlzylindervollkatalysator der Geometrie 5 mm x 3 mm x 2 mm bzw. 5 mm x 2 mm x 2 mm (jeweils Außendurchmesser x Höhe x Innendurchmesser) sowie der Multimetalloxid II - Vollkatalysator gemäß Beispiel 1 der DE-A 197 46 210. Ferner wären die Multimetalloxid-Katalysatoren der US-A 4,438,217 zu nennen. Letzteres gilt insbesondere dann, wenn diese eine Hohlzylinder-Geometrie der Ausmaße 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Ebenso eignen sich die Multimetalloxidkatalysatoren und Geometrien der DE-A 101 01 695 bzw. WO 02/062737.

Weiterhin sind das Beispiel 1 aus der DE-A 100 46 957 (Stöchiometrie: $[Bi_2W_2O_9 \times 2WO_3]_{0,5} \cdot [Mo_{12}Co_{5,6}Fe_{2,94}Si_{1,59}Ko_{0,08}O_x]_1$) als Hohlzylinder (Ring) vollkatalysator der Geometrie 5 mm x 3 mm x 2 mm bzw. 5 mm x 2 mm x 2 mm (jeweils Außendurchmesser x Länge x Innendurchmesser), sowie die Schalenkatalysatoren 1, 2 und 3 aus der DE-A 100 63 162 (Stöchiometrie: $Mo_{12}Bi_{1,0}Fe_3Co_7Si_{1,6}K_{0,08}$), jedoch als ringförmige Schalenkatalysatoren entsprechender Schalendicke und auf Trägerringe der Geometrie 5 mm x 3 mm x 1,5 mm bzw. 7 mm x 3 mm x 1,5 mm (jeweils Außendurchmesser x Länge x Innendurchmesser) aufgebracht, geeignet.

Eine Vielzahl der für die Katalysatoren der (frischen) Festbettkatalysatorschüttung einer erfindungsgemäßen Propylenpartialoxidation zu Acrolein geeigneten Multimetalloxidaktivmassen lässt sich unter der allgemeinen Formel I,

$$Mo_{12}Bi_aFe_bX^1_cX^2_dX^3_eX^4_fO_n \qquad (I),$$

in der die Variablen nachfolgende Bedeutung aufweisen:

$X^1$ =   Nickel und/oder Kobalt,
$X^2$ =   Thallium, ein Alkalimetall und/oder ein Erdalkalimetall,
$X^3$ =   Zink, Phosphor, Arsen, Bor, Antimon, Zinn, Cer, Blei und/oder Wolfram,
$X^4$ =   Silicium, Aluminium, Titan und/oder Zirkonium,

a =   0,5 bis 5,
b =   0,01 bis 5, vorzugsweise 2 bis 4,
c =   0 bis 10, vorzugsweise 3 bis 10,
d =   0 bis 2, vorzugsweise 0,02 bis 2,
e =   0 bis 8, vorzugsweise 0 bis 5,
f =   0 bis 10 und
n =   eine Zahl, die durch die Wertigkeit und Häufigkeit der von Sauerstoff verschiedenen Elemente in I bestimmt wird,

subsumieren.

Sie sind in an sich bekannter Weise erhältlich (siehe z. B. die DE-A 40 23 239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel I in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemisch aus Inertgas, $NH_3$, CO und/oder $H_2$) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oderAmmoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150 °C erfolgt.

Üblicherweise werden die Multimetalloxidaktivmassen der allgemeinen Formel I in der (frischen) Festbettkatalysatorschüttung für eine erfindungsgemäße Propylenpartialoxidation zu Acrolein nicht in Pulverform sondern zu bestimmten Katalysatorgeometrien geformt eingesetzt, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 29 09 671, der EP-A 293 859 oder aus der EP-A 714 700 bekannt ist. Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 $\mu$m, bevorzugt im Bereich 50 bis 500 $\mu$m und besonders bevorzugt im Bereich 150 bis 250 $\mu$m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren in der ersten Reaktionsstufe unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder, bevorzugt werden. Geeignet ist die Verwendung von im wesentlichen unporösen, oberflächenrauhen, kugelförmigen Trägern aus Steatit (z. B. Steatit C 220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für die (frischen) Katalysatoren einer erfindungsgemäßen Partialoxidation von Propylen zu Acrolein geeignete Multimetalloxidaktivmassen sind ferner Massen der allgemeinen Formel II,

$$[Y^1_{a'}Y^2_{b'}O_{x'}]_p[Y^3_{c'}Y^4_{d'}Y^5_{e'}Y^6_{f'}Y^7_{g'}Y^2_{h'}O_{y'}]_q \qquad (II),$$

in der die Variablen folgende Bedeutung haben:

$Y^1$ = nur Wismut oder Wismut und wenigstens eines der Elemente Tellur, Antimon, Zinn und Kupfer,
$Y^2$ = Molybdän oder Molybdän und Wolfram,
$Y^3$ = ein Alkalimetall, Thallium und/oder Samarium,
$Y^4$ = ein Erdalkalimetall, Nickel, Kobalt, Kupfer, Mangan, Zink, Zinn, Cadmium und/oder Quecksilber,
$Y^5$ = Eisen oder Eisen und wenigstens eines der Elemente Chrom und Cer,
$Y^6$ = Phosphor, Arsen, Bor und/oder Antimon,
$Y^7$ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

$a'$ = 0,01 bis 8,
$b'$ = 0,1 bis 30,
$c'$ = 0 bis 4,
$d'$ = 0 bis 20,
$e' >$ 0 bis 20,
$f'$ = 0 bis 6,
$g'$ = 0 bis 15,
$h'$ = 8 bis 16,
$x',y'$ = Zahlen, die durch die Wertigkeit und Häufigkeit der von Sauerstoff verschiedenen Elemente in II bestimmt werden und
$p,q$ = Zahlen, deren Verhältnis $p/q$ 0,1 bis 10 beträgt,

enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Besonders vorteilhafte erfindungsgemäße Multimetalloxidmassen II sind solche, in denen $Y^1$ nur Wismut ist.

Unter diesen werden wiederum jene bevorzugt, die der allgemeinen Formel III,

$$[Bi_{a''}Z^2_{b''}O_{x''}]_{p''} [Z^2_{12}Z^3_{c''}Z^4_{d''}Fe_{e''}Z^5_{f''}Z^6_{g''}Z^7_{h''}O_{y''}]_{q''} \qquad (III),$$

in der die Varianten folgende Bedeutung haben:

$Z^2$ = Molybdän oder Molybdän und Wolfram,
$Z^3$ = Nickel und/oder Kobalt,
$Z^4$ = Thallium, ein Alkalimetall und/oder ein Erdalkalimetall,
$Z^5$ = Phosphor, Arsen, Bor, Antimon, Zinn, Cer und/oder Blei,
$Z^6$ = Silicium, Aluminium, Titan und/oder Zirkonium,
$Z^7$ = Kupfer, Silber und/oder Gold,

$a''$ = 0,1 bis 1,
$b''$ = 0,2 bis 2,
$c''$ = 3 bis 10,
$d''$ = 0,02 bis 2,
$e''$ = 0,01 bis 5, vorzugsweise 0,1 bis 3,
$f''$ = 0 bis 5,
$g''$ = 0 bis 10,
$h''$ = 0 bis 1,
$x'',y''$ = Zahlen, die durch die Wertigkeit und Häufigkeit der von Sauerstoff verschiedenen Element in III bestimmt werden,
$p'',q''$ = Zahlen, deren Verhältnis $p''/q''$ 0,1 bis 5, vorzugsweise 0,5 bis 2 beträgt,

entsprechen, wobei diejenigen Massen III ganz besonders bevorzugt werden, in denen $Z^2_{b''}$ = (Wolfram)$_{b''}$ und $Z^2_{12}$ =

(Molybdän)$_{12}$ ist.

Ferner ist es von Vorteil, wenn wenigstens 25 mol% (bevorzugt wenigstens 50 mol-% und besonders bevorzugt wenigstens 100 mol-%) des gesamten Anteils $[Y^1{}_{a'}Y^2{}_{b'}O_{x'}]_p$ ($[Bi_{a''}Z^2{}_{b''}O_{x''}]_{p''}$) der erfindungsgemäß geeigneten Multimetalloxidmassen II (Multimetalloxidmassen III) in den erfindungsgemäß geeigneten Multimetalloxidmassen II (Multimetalloxidmassen III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1{}_{a'}Y^2{}_{b'}O_{x'}$ ($[Bi_a{}''Z^2{}_{b''}O_{x''}$) vorliegen, deren Größtdurchmesser im Bereich 1 nm bis 100 μm liegt.

Hinsichtlich der Formgebung gilt bezüglich Multimetalloxidmassen II-Katalysatoren das bei den Multimetalloxidmassen I-Katalysatoren Gesagte.

Die Herstellung von Multimetalloxidmassen II-Aktivmassen ist z. B. in der EP-A 575 897 sowie in der DE-A 198 55 913 beschrieben.

Die vorstehend empfohlenen inerten Trägermaterialien kommen u.a. auch als inerte Materialien zur Verdünnung und/oder Abgrenzung des entsprechenden Katalysatorfestbetts, bzw. als deren schützende Vorschüttung in Betracht.

Für die (frischen) Katalysatoren einer erfindungsgemäßen Partialoxidation von Acrolein zu Acrylsäure kommen prinzipiell alle Mo und V enthaltenden Multimetalloxidmassen als Aktivmassen in Betracht, z. B. jene der DE-A 100 46 928.

Eine Vielzahl derselben, z. B. diejenigen der DE-A 198 15 281, lässt sich unter der allgemeinen Formel IV,

$$Mo_{12}V_aX^1{}_bX^2{}_cX^3{}_dX^4{}_eX^5{}_fX^6{}_gO_n \qquad (IV),$$

in der die Variablen folgende Bedeutung haben:

$X^1 =$      W, Nb, Ta, Cr und/oder Ce,
$X^2 =$      Cu, Ni, Co, Fe, Mn und/oder Zn,
$X^3 =$      Sb und/oder Bi,
$X^4 =$      eines oder mehrere Alkalimetalle,
$X^5 =$      eines oder mehrere Erdalkalimetalle,
$X^6 =$      Si, Al, Ti und/oder Zr,

$a =$      1 bis 6,
$b =$      0,2 bis 4,
$c =$      0,5 bis 18,
$d =$      0 bis 40,
$e =$      0 bis 2,
$f =$      0 bis 4,
$g =$      0 bis 40 und
$n =$      eine Zahl, die durch die Wertigkeit und Häufigkeit der von Sauerstoff verschiedenen Elemente in IV bestimmt wird,

subsumieren.

Erfindungsgemäß bevorzugte Ausführungsformen innerhalb der aktiven Multimetalloxide IV sind jene, die von den nachfolgenden Bedeutungen der Variablen der allgemeinen Formel IV erfasst werden:

$X^1 =$      W, Nb, und/oder Cr,
$X^2 =$      Cu, Ni, Co, und/oder Fe,
$X^3 =$      Sb,
$X^4 =$      Na und/oder K,
$X^5 =$      Ca, Sr und/oder Ba,
$X^6 =$      Si, Al, und/oder Ti,

$a =$      1,5 bis 5,
$b =$      0,5 bis 2,
$c =$      0,5 bis 3,
$d =$      0 bis 2,
$e =$      0 bis 0,2,
$f =$      0 bis 1 und
$n =$      eine Zahl, die durch die Wertigkeit und Häufigkeit der von Sauerstoff verschiedenen Elemente in IV bestimmt wird.

Erfindungsgemäß ganz besonders bevorzugte Multimetalloxide IV sind jedoch jene der allgemeinen Formel V,

$$Mo_{12}V_{a'}Y^1_{b'}Y^2_{c'}Y^5_{f'}Y^6_{g'}O_{n'} \qquad (V),$$

mit

$Y^1 =$     W und/oder Nb,
$Y^2 =$     Cu und/oder Ni,
$Y^5 =$     Ca und/oder Sr,
$Y^6 =$     Si und/oder Al,

$a' =$     2 bis 4,
$b' =$     1 bis 1,5,
$c' =$     1 bis 3,
$f' =$     0 bis 0,5,
$g' =$     0 bis 8 und
$n' =$     eine Zahl, die durch die Wertigkeit und Häufigkeit der von Sauerstoff verschiedenen Elementen in V bestimmt wird.

Die erfindungsgemäß geeigneten Multimetalloxidaktivmassen (IV) sind in an sich bekannter, z. B. in der DE-A 43 35 973 oder in der EP-A 714 700 offenbarter, Weise erhältlich.

Prinzipiell können für die Katalysatoren der frischen Festbettkatalysatorschüttung einer erfindungsgemäßen Acroleinpartialoxidation zu Acrylsäure geeignete Multimetalloxidaktivmassen, insbesondere solche der allgemeinen Formel IV, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemische aus Inertgas und reduzierenden Gasen wie $H_2$, $NH_3$, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150 °C erfolgt.

Die resultierenden Multimetalloxidmassen, insbesondere jene der allgemeinen Formel IV, werden in der Regel nicht in Pulverform sondern zu bestimmten Katalysatorgeometrien geformt in der (frischen) Festbettkatalysatorschüttung für die erfindungsgemäße Partialoxidation von Acrolein zu Acrylsäure eingesetzt, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 29 09 671, der EP-A 293 859 oder aus der EP-A 714 700 bekannt ist.

Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 $\mu$m, bevorzugt im Bereich 50 bis 500 $\mu$m und

besonders bevorzugt im Bereich 150 bis 250 $\mu$m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder mit Splittauflage, bevorzugt werden. Geeignet ist die Verwendung von im wesentlichen unporösen, oberflächenrauhen, kugelförmigen Trägern aus Steatit (z. B. Steatit C 220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für die Katalysatoren einer (frischen) Festbettkatalysatorschüttung zur erfindungsgemäßen Acroleinpartialoxidation zu Acrylsäure zu verwendende Multimetalloxidaktivmassen sind ferner Massen der allgemeinen Formel VI,

$$[D]_p[E]_q \qquad (VI),$$

in der die Variablen folgende Bedeutung haben:

$D =$ $Mo_{12}V_{a''}Z^1_{b''}Z^2_{c''}Z^3_{d''}Z^4_{e''}Z^5_{f''}Z^6_{g''}O_{x''}$,
$E =$ $Z^7_{12}Cu_{h''}H_{i''}O_{y''}$,
$Z^1 =$ W, Nb, Ta, Cr und/oder Ce,
$Z^2 =$ Cu, Ni, Co, Fe, Mn und/oder Zn,
$Z^3 =$ Sb und/oder Bi,
$Z^4 =$ Li, Na, K, Rb, Cs und/oder H,
$Z^5 =$ Mg, Ca, Sr und/oder Ba,
$Z^6 =$ Si, Al, Ti und/oder Zr,
$Z^7 =$ Mo, W, V, Nb und/oder Ta, vorzugsweise Mo und/oder W,

$a'' =$ 1 bis 8,
$b'' =$ 0,2 bis 5,
$c'' =$ 0 bis 23,
$d'' =$ 0 bis 50,
$e'' =$ 0 bis 2,
$f'' =$ 0 bis 5,
$g'' =$ 0 bis 50,
$h'' =$ 4 bis 30,
$i'' =$ 0 bis 20 und
$x'',y'' =$ Zahlen, die durch die Wertigkeit und Häufigkeit der von Sauerstoff verschiedenen Element in VI bestimmt werden und
$p,q =$ von Null verschiedene Zahlen, deren Verhältnis p/q 160:1 bis 1:1 beträgt,

und die dadurch erhältlich sind, dass man eine Multimetalloxidmasse E

$$Z^7_{12}Cu_{h''}H_{i''}O_{y''} \qquad (E),$$

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, $Z^1$, $Z^2$, $Z^3$, $Z^4$, $Z^5$, $Z^6$, die die vorgenannten Elemente in der Stöchiometrie D

$$Mo^{12}V_{a''}Z^1_{b''}Z^2_{c''}Z^3_{d''}Z^4_{e''}Z^5_{f''}Z^6_{g''} \qquad (D),$$

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600 °C calciniert.

Bevorzugt sind die Multimetalloxidmassen VI, bei denen die Einarbeitung der vorgebildeten festen Ausgangsmasse 1 in eine wässrige Ausgangsmasse 2 bei einer Temperatur < 70 °C erfolgt. Eine detaillierte Beschreibung der Herstellung von Multimetalloxidmassen VI-Katalysatoren enthalten z. B. die EP-A 668 104, die DE-A 197 36 105, die DE-A 100 46 928, die DE-A 197 40 493 und die DE-A 195 28 646.

Hinsichtlich der Formgebung gilt bezüglich Multimetalloxidmassen VI-Katalysatoren das bei den Multimetalloxidmassen IV-Katalysatoren Gesagte.

Darüber hinaus eignen sich als für die (frischen) Katalysatoren einer Festbettkatalysatorschüttung zur erfindungsgemäßen Partialoxidation von Acrolein zu Acrylsäure geeignete Multimetalloxidmassen jene der DE-A 198 15 281, insbesondere alle beispielhaften Ausführungsformen aus dieser Schrift. Hinsichtlich der Formgebung gilt das vorstehend Gesagte.

Für die (frische) Festbettkatalysatorschüttung eines erfindungsgemäßen Verfahrens zur Partialoxidation von Acrolein zu Acrylsäure besonders geeignete Katalysatoren sind die Schalenkatalysatoren S1 (Stöchiometrie: $Mo_{12}V_3W_{1,2}Cu_{2,4}O_n$) und S7 (Stöchiometrie: $Mo_{12}V_3W_{1,2}Cu_{1,6}Ni_{0,8}O_n$) aus der DE-A 4442346 mit einem Aktivmassenanteil von 27 Gew.-% und einer Schalendicke von 230 $\mu$m, der Schalenkatalysator aus Herstellungsbeispiel 5 der DE-A 100 46 928 (Stöchiometrie: $Mo_{12}V_3W_{1,2}Cu_{2,4}O_n$) mit einem Aktivmassenanteil von 20 Gew.-%, die Schalenkatalysatoren gemäß den Beispielen 1 bis 5 aus der DE-A 198 15 281, jedoch ebenso wie die vorstehend genannten Schalenkatalysatoren für die zweite Reaktionsstufe auf Trägerringe der Geometrie 7 mm x 3 mm x 4 mm (Außendurchmesser x Länge x Innendurchmesser) mit einem Aktivmassenanteil von 20 Gew.-% (bezogen auf die Gesamtmasse des Schalenkatalysators) aufgebracht, sowie ein Schalenkatalysator mit zweiphasiger Aktivmasse der Stöchiometrie $(Mo_{10,4}V_3W_{1,2}O_x)$ $(CuMo_{0,5}W_{0,5}O_4)_{1,6}$, und hergestellt gemäß der DE-A 19736105 und einem Aktivmassenanteil von 20 Gew.-% auf den vorgenannten 7 mm x 3 mm x 4 mm Träger aufgebracht.

Die vorstehend für eine erfindungsgemäße Acroleinpartialoxidation zu Acrylsäure empfohlenen (frischen) Katalysatoren sind für diese Reaktion aber auch dann geeignet, wenn man alles beibehält und nur die Trägergeometrie auf 5 mm x 3 mm x 1,5 mm abändert (Außendurchmesser x Länge x Innendurchmesser). Ferner können die genannten Multimetalloxide aber auch in Form der entsprechenden Vollkatalysatorringe als Katalysatoren für eine solche Partialoxidation eingesetzt werden.

Prinzipiell kann bei einer erfindungsgemäßen Partialoxidation von Propylen zu Acrolein die volumenspezifische Aktivität des (frischen) Katalysatorfestbetts in Strömungsrichtung des Reaktionsgasgemischs über die Länge des Strömungsweges (d.h., über die Länge des Katalysatorfestbetts) konstant sein, oder vorteilhaft wenigstens einmal (kontinuierlich, oder abrupt oder stufenförmig) zunehmen. Dabei ist von Vorteil, wenn sich die Aktivmassenzusammensetzung über die Länge des Strömungsweges des Reaktionsgasgemischs (d.h. innerhalb des frischen Katalysatorfestbetts) nicht ändert. Im folgenden soll das (frische) Katalysatorfestbett für eine erfindungsgemäße heterogen katalysierte Gasphasen-Partialoxidation von Propylen zu Acrolein als Katalysatorfestbett 1 bzw. als Festbettkatalysatorschüttung 1 bezeichnet werden. In entsprechender Weise wird das zugehörige Reaktionsgasgemisch als Reaktionsgasgemisch 1 bzw. als Reaktionsgaseingangsgemisch 1 bezeichnet.

Erfindungsgemäß vorteilhaft ist es, wenn die Festbettkatalysatorschüttung 1 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 1 beim Übergang von einer Festbettkatalysatorschüttungszone in eine andere Festbettkatalysatorschüttungszone sprunghaft zunimmt.

Die volumenspezifische (d.h., die auf die Einheit der jeweiligen Schüttungsvolumens normierte) Aktivität einer Festbettkatalysatorschüttungszone kann nun in über die Festbettkatalysatorschüttungszone im wesentlichen konstanter Weise dadurch eingestellt werden, dass man von einer Grundmenge einheitlich hergestellter Katalysatorformkörper ausgeht (ihre Schüttung entspricht der maximal erzielbaren volumenspezifischen Aktivität) und diese in der jeweiligen Festbettkatalysatorschüttungszone mit sich bezüglich der heterogen katalysierten partiellen Gasphasenoxidation im wesentlichen inert verhaltenden Formkörpern (Verdünnungsformkörper) homogen verdünnt. Je höher der Anteil der Verdünnungsformkörper gewählt wird, desto geringer ist die in einem bestimmten Volumen der Schüttung enthaltene Aktivmasse bzw. Katalysatoraktivität. Als Materialien für solche inerten Verdünnungsformkörper kommen prinzipiell alle diejenigen in Betracht, die sich auch als Trägermaterial für erfindungsgemäß geeignete Schalenkatalysatoren eignen.

Als solche Materialien kommen z. B. poröse oder unporöse Aluminiumoxide, Siliciumdioxid, Thoriumdioxid, Zirkondioxid, Siliziumcarbid, Silikate wie Magnesium -oder Aluminiumsilikat oder der bereits erwähnte Steatit (z. B. Steatit C 220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

Erfindungsgemäß günstig ist es, wie bereits gesagt, wenn sich die chemische Zusammensetzung der verwendeten Aktivmasse über die gesamte Festbettkatalysatorschüttung 1 nicht verändert. D.h., die für einen einzelnen Katalysatorformkörper verwendete Aktivmasse kann zwar ein Gemisch aus verschiedenen, z. B. die Elemente Mo, Fe und Bi enthaltenden, Multimetalloxiden sein, für alle Katalysatorformkörper der Festbettkatalysatorschüttung 1 ist dann jedoch das gleiche Gemisch zu verwenden.

Eine in Strömungsrichtung des Reaktionsgasgemisches 1 über die Festbettkatalysatorschüttung zonenweise zunehmende (ist besonders vorteilhaft) volumenspezifische Aktivität lässt sich somit in einfacher Weise z. B. dadurch einstellen, dass man die Schüttung in einer ersten Festbettkatalysatorschüttungszone mit einem hohen Anteil an inerten Verdünnungsverformkörpern bezogen auf eine Sorte von Katalysatorformkörpern beginnt, und dann diesen Anteil an Verdünnungsformkörpern in Strömungsrichtung zonenweise verringert.

Eine solche erfindungsgemäß vorteilhafte zonenweise Zunahme der volumenspezifischen Aktivität ist aber auch z. B. dadurch möglich, dass man bei gleich bleibender Geometrie und Aktivmassenart eines Schalenkatalysatorformkörpers zonenweise die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse zonenweise den Anteil an Katalysatorformkörpern mit höherem Aktivmassenanteil steigert. Alternativ kann man auch die Aktivmassen selbst verdünnen, in dem man bei der Aktivmassenherstellung z. B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten.

Je mehr verdünnend wirkendes Material zugesetzt wird, desto geringer wird die resultierende Aktivität sein. Analoge Wirkung lässt sich z. B. auch dadurch erzielen, dass man bei Mischungen aus Vollkatalysatoren und aus Schalenkatalysatoren (bei identischer Aktivmasse) in entsprechender Weise das Mischungsverhältnis verändert. Des weiteren lässt sich eine Variation der volumenspezifischen Aktivität durch den Einsatz von Katalysatorgeometrien mit unterschiedlicher Schüttdichte erzielen (z. B. bei Vollkatalysatoren mit identischer Aktivmassenzusammensetzung der verschiedenen Geometrien). Selbstredend lassen sich die beschriebenen Varianten auch kombiniert anwenden.

Natürlich können für die Festbettkatalysatorschüttung 1 aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum zonenweise in ihrer Zusammensetzung variiert und/oder mit unterschiedlichen Mengen inerter Verdünnungsformkörper verdünnt werden, so dass die volumenspezifische Aktivität in Strömungsrichtung des Reaktionsgasgemischs zonenweise zunimmt.

Vorab und/oder im Anschluss an die Festbettkatalysatorschüttung 1 können sich ausschließlich aus Inertmaterial (z. B. nur Verdünnungsformkörpern) bestehende Schüttungen befinden (sie werden in dieser Schrift begrifflich nicht der Festbettkatalysatorschüttung 1 zugerechnet, da sie keine Formkörper enthalten, die Multimetalloxidaktivmasse aufweisen). Dabei können die für die Inertschüttung verwendeten Verdünnungsformkörper die gleiche Geometrie wie die in der Festbettkatalysatorschüttung 1 verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z. B. kugelförmig anstelle ringförmig).

Häufig weisen die für solche Inertschüttungen verwendeten Formkörper die ringförmige Geometrie 7 mm x 7 mm x 4 mm bzw. 7 mm x 3 mm x 4 mm (jeweils Außendurchmesser x Länge x Innendurchmesser) oder die kugelförmige Geometrie mit dem Durchmesser d = 4 - 5 mm auf. Die Temperaturzonen A und B können sich beim erfindungsgemäßen Verfahren auch auf die Inertschüttungen erstrecken. Erfindungsgemäß vorteilhaft erfassen sowohl die Temperaturzone A als auch die Temperaturzone B für eine erfindungsgemäße Partialoxidation von Propylen zu Acrolein jeweils nicht mehr als drei Festbettkatalysatorschüttungszonen (erfindungsgemäß vorteilhaft wird wenigstens eine Festbettkatalysatorschüttungszone von beiden Temperaturzonen erfasst).

Erfindungsgemäß besonders bevorzugt umfasst die gesamte Festbettkatalysatorschüttung 1 nicht mehr als fünf, zweckmäßig nicht mehr als vier bzw. drei Festbettkatalysatorschüttungszonen.

Beim Übergang von einer Festbettkatalysatorschüttungszone in eine andere Festbettkatalysatorschüttungszone (in Strömungsrichtung des Reaktionsgasgemisches 1) der Festbettkatalysatorschüttung 1 sollte (bei einheitlicher Aktivmasse über die gesamte Festbettkatalysatorschüttung 1) die volumenspezifische Aktivmasse (d.h., das Gewicht der im Einheitsschüttungsvolumen enthaltenen Multimetalloxidaktivmasse) erfindungsgemäß zweckmäßig um wenigstens 5 Gew.-%, bevorzugt um wenigstens 10 Gew.-% zunehmen (dies gilt insbesondere auch bei einheitlichen Katalysatorformkörpern über die gesamte Festbettkatalysatorschüttung 1). In der Regel wird diese Zunahme beim erfindungsgemäßen Verfahren einer heterogen katalysierten Partialoxidation von Propylen zu Acrolein nicht mehr als 50 Gew.-%, meist nicht mehr als 40 Gew.-% betragen. Ferner sollte bei einheitlicher Aktivmasse über die gesamte Festbettkatalysatorschüttung 1 der Unterschied in der volumenspezifischen Aktivmasse derjenigen Festbettkatalysatorschüttungszone mit der geringsten volumenspezifischen Aktivität und derjenigen Festbettkatalysatorschüttungszone mit der höchsten volumenspezifischen Aktivität erfindungsgemäß vorteilhaft nicht mehr als 50 Gew.-%, vorzugsweise nicht mehr als 40 Gew.-% und in der Regel nicht mehr als 30 Gew.-% betragen.

Häufig wird bei einem erfindungsgemäßen Verfahren der heterogen katalysierten Partialoxidation von Propylen zu Acrolein die Festbettkatalysatorschüttung 1 aus nur zwei Festbettkatalysatorschüttungszonen bestehen.

Erfindungsgemäß bevorzugt ist die in Strömungsrichtung des Reaktionsgasgemisches 1 letzte Festbettkatalysatorschüttungszone der Festbettkatalysatorschüttung 1 unverdünnt. D.h., sie besteht vorzugsweise ausschließlich aus Katalysatorformkörpern. Im Bedarfsfall kann sie auch aus einer Schüttung aus Katalysatorformkörpern bestehen, deren volumenspezifische Aktivität z. B. durch Verdünnung mit Inertmaterial abgesenkt, z. B. um 10 %, ist

Besteht die Festbettkatalysatorschüttung 1 für eine heterogen katalysierte Partialoxidation von Propylen zu Acrolein nur aus zwei Festbettkatalysatorschüttungszonen, ist es erfindungsgemäß in der Regel vorteilhaft, wenn die Festbettkatalysatorschüttungszone mit der höchsten volumenspezifischen Aktivität nicht bis in die Temperaturzone A hineinragt (insbesondere dann, wenn in der Temperaturzone A und in der Temperaturzone B die Temperierung mittels eines fließenden Wärmeträgers erfolgt, der jeweils im Gegenstrom (über den Reaktor betrachtet) zum Reaktionsgasgemisch 1 strömt). D.h., in günstiger Weise wird die Festbettkatalysatorschüttungszone mit der geringeren volumenspezifischen Aktivität in die Temperaturzone B hineinragen und die Festbettkatalysatorschüttungszone mit der höheren volumenspezifischen Aktivität in der Temperaturzone B beginnen und enden (d.h., hinter dem Übergang von der Temperaturzone A in die Temperaturzone B ihren Anfang haben).

Insbesondere bei Belastungen der Festbettkatalysatorschüttung 1 mit Propylen im Bereich von 100 bis 160 Nl/l·h und/oder bei Mitverwendung von, bezogen auf das Reaktionsgaseingangsgemisch 1, z. B. bis zu 50 Vol.-% Propan als inertem Verdünnungsgas, hat es sich jedoch als zweckmäßig erwiesen, die Festbettkatalysatorschüttungszone mit der höchsten volumenspezifischen Aktivität in die Temperaturzone A hineinragen zu lassen. Dies insbesondere bei Gegenstromfahrweise (über den Reaktor betrachtet) von Salzbädern und Reaktionsgasgemisch.

Besteht die Festbettkatalysatorschüttung 1 nur aus drei Festbettkatalysatorschüttungszonen, ist es erfindungsgemäß in der Regel gleichfalls vorteilhaft, wenn die Festbettkatalysatorschüttungszone mit der höheren volumenspezifischen Aktivität nicht bis in die Temperaturzone A hineinragt sondern in der Temperaturzone B beginnt und endet, d.h., hinter dem Übergang von der Temperaturzone A in die Temperaturzone B ihren Anfang hat (insbesondere dann, wenn in der Temperaturzone A und in der Temperaturzone B die Temperierung mittels eines fließenden Wärmeträgers erfolgt, der jeweils im Gegenstrom zum Reaktionsgasgemisch 1 strömt). D.h., normalerweise wird in diesem Fall die Festbettkatalysatorschüttungszone mit der zweithöchsten volumenspezifischen Aktivität sowohl in die Temperaturzone A als auch in die Temperaturzone B hineinragen.

Besteht die Festbettkatalysatorschüttung 1 aus vier Festbettkatalysatorschüttungszonen, ist es erfindungsgemäß in der Regel vorteilhaft, wenn die Festbettkatalysatorschüttungszone mit der dritthöchsten volumenspezifischen Aktivität sowohl in die Temperaturzone A als auch in die Temperaturzone B hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch 1 strömt).

Im Fall einer Gleichstromführung von Reaktionsgasgemisch 1 und Wärmeträgern in den Temperaturzone A und B kann es vorteilhaft sein, wenn beim erfindungsgemäßen Verfahren innerhalb der Festbettkatalysatorschüttung 1 die Festbettkatalysatorschüttungszone mit der höchstens volumenspezifischen Aktivität in die Temperaturzone A hineinragt.

Generell lässt sich die volumenspezifische Aktivität zwischen zwei Festbettkatalysatorschüttungszonen einer Festbettkatalysatorschüttung 1 experimentell in einfacher Weise so differenzieren, dass unter identischen Randbedingungen (vorzugsweise den Bedingungen des ins Auge gefassten Verfahrens) über Festbettkatalysatorschüttungen derselben Länge, aber jeweils der Zusammensetzung der jeweiligen Festbettkatalysatorschüttungszone entsprechend, das gleich Propen enthaltende Reaktionsgasgemisch geführt wird. Die höhere umgesetzte Menge an Propen weist die höhere volumenspezifische Aktivität aus.

Beträgt die Gesamtlage der Festbettkatalysatorschüttung 1 $L^1$, ist es erfindungsgemäß vorteilhaft, wenn sich im Bereich $X^1 \pm L^1 \dfrac{4}{100}$ bzw. im Bereich $X^1 \pm L^1 \dfrac{3}{100}$ bzw. im Bereich $X^1 \pm L^1 \dfrac{2}{100}$ kein Übergang von einer Festbettkatalysatorschüttungszone in eine andere Festbettkatalysatorschüttungszone befindet, wobei X der Ort (die Stelle) innerhalb der Festbettkatalysatorschüttung 1 ist, an dem der Übergang von der Temperaturzone A in die Temperaturzone B erfolgt.

Erfindungsgemäß bevorzugt ist beim erfindungsgemäßen Verfahren die Festbettkatalysatorschüttung 1 in Strömungsrichtung des Reaktionsgasgemisches 1 wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h. z. B. auf einer Länge von 0,70 bis 1,50 m, bevorzugt 0,90 bis 1,20 m), jeweils der Gesamtlänge der Festbettkatalysatorschüttung 1, ein homogenes Gemisch aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diese erste Zone der Festbettkatalysatorschüttung 1 befindet sich dann erfindungsgemäß vorteilhaft bis zum Ende der Länge der Festbettkatalysatorschüttung 1 (d.h., z.B.

auf einer Länge von 1,00 (bzw. 1,40 m) bis 3,00 m, bevorzugt 2,00 bis 3,00 m) entweder eine nur in geringerem Umfang (als in der ersten Zone) verdünnte Schüttung der Katalysatorformkörper, oder, ganz besonders bevorzugt, eine alleinige (unverdünnte) Schüttung derselben Katalysatorformkörper, die auch in der ersten Zone verwendet worden sind. Das Vorgenannte trifft insbesondere dann zu, wenn in der Festbettkatalysatorschüttung 1 als Katalysatorformkörper Vollkatalysatorringe oder Schalenkatalysatorringe (insbesondere jene, die in dieser Schrift als bevorzugt genannt werden) eingesetzt werden. Mit Vorteil weisen im Rahmen der vorgenannten Strukturierung sowohl die Katalysatorformkörper als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

Das Vorgenannte trifft auch dann zu, wenn anstelle inerter Verdünnungsformkörper Schalenkatalysatorformkörper verwendet werden, deren Aktivmassenanteil um 2 bis 15 Gew.-% Punkte tiefer liegt, als der Aktivmassenanteil der am Ende der Festbettkatalysatorschüttung 1 gegebenenfalls verwendeten Schalenkatalysatorformkörper.

Eine reine Inertmaterialschüttung, deren Länge, bezogen auf die Länge der Festbettkatalysatorschüttung 1, zweckmäßig 5 bis 20 % beträgt, leitet in Strömungsrichtung des Reaktionsgasgemisches in der Regel die Festbettkatalysatorschüttung 1 ein. Sie wird normalerweise als Aufheizzone für das Reaktionsgasgemisch 1 genutzt.

Erfindungsgemäß vorteilhaft erstreckt sich nun bei den vorgenannten Festbettkatalysatorschüttungen 1 die Festbettkatalysatorschüttungszone mit der geringeren volumenspezifischen Aktivität noch auf 5 bis 20 %, häufig auf 5 bis 15 % ihrer Länge in die Temperaturzone B.

Erfindungsgemäß zweckmäßig erstreckt sich die Temperaturzone A auch auf eine zur Festbettkatalysatorschüttung 1 gegebenenfalls angewandte Vorschüttung aus Inertmaterial.

Prinzipiell kann bei einer erfindungsgemäßen Partialoxidation von Acrolein zu Acrylsäure die volumenspezifische Aktivität des (frischen) Katalysatorfestbetts in Strömungsrichtung des Reaktionsgasgemischs über die Länge des Strömungsweges (d.h., über die Länge des Katalysatorfestbetts) konstant sein, oder vorteilhaft wenigstens einmal (kontinuierlich, oder abrupt, oder stufenförmig) zunehmen. Dabei ist es von Vorteil, wenn sich die Aktivmassenzusammensetzung über die Länge des Strömungsweges des Reaktionsgasgemischs (d.h., innerhalb des frischen Katalysatorfestbetts) nicht ändert. Im folgenden soll das (frische) Katalysatorfestbett für eine erfindungsgemäße heterogen katalysierte Gasphasen-Partialoxidation von Acrolein zu Acrylsäure als Katalysatorfestbett 2 bzw. als Festbettkatalysatorschüttung 2 bezeichnet werden. In entsprechender Weise wird das zugehörige Reaktionsgasgemisch als Reaktionsgasgemisch 2 bzw. als Reaktionsgaseingangsgemisch 2 bezeichnet.

Erfindungsgemäß vorteilhaft ist es, wenn die Festbettkatalysatorschüttung 2 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 2 beim Übergang von einer Festbettkatalysatorschüttungszone in eine andere Festbettkatalysatorschüttungszone sprunghaft zunimmt.

Die volumenspezifische (d.h., die auf die Einheit des jeweiligen Schüttungsvolumens normierte) Aktivität einer Festbettkatalysatorschüttungszone kann nun in über die Festbettkatalysatorschüttungszone im wesentlichen konstanter Weise dadurch eingestellt werden, dass man von einer Grundmenge einheitlich hergestellter Katalysatorformkörper ausgeht (ihre Schüttung entspricht der maximal erzielbaren volumenspezifischen Aktivität) und diese in der jeweiligen Festbettkatalysatorschüttungszone mit sich bezüglich der heterogen katalysierten partiellen Gasphasenoxidation im wesentlichen inert verhaltenden Formkörpern (Verdünnungsformkörper) homogen verdünnt. Je höher der Anteil der Verdünnungsformkörper gewählt wird, desto geringer ist die in einem bestimmten Volumen der Schüttung enthaltene Aktivmasse bzw. Katalysatoraktivität. Als Materialien für solche inerten Verdünnungsformkörper kommen prinzipiell alle diejenigen in Betracht, die sich auch als Trägermaterial für erfindungsgemäß geeignete Schalenkatalysatoren eignen.

Als solche Materialien kommen z. B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z. B. Steatit C 220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

Erfindungsgemäß günstig ist es, wie bereits gesagt, wenn sich die chemische Zusammensetzung der verwendeten Aktivmasse über die gesamte Festbettkatalysatorschüttung 2 nicht verändert. D.h., die für einen einzelnen Katalysatorformkörper verwendete Aktivmasse kann zwar ein Gemisch aus verschiedenen, z. B. die Elemente Mo und V enthaltenden, Multimetalloxiden sein, für alle Katalysatorformkörper der Festbettkatalysatorschüttung 2 ist dann jedoch das gleiche Gemisch zu verwenden.

Eine in Strömungsrichtung des Reaktionsgasgemisches 2 über die Festbettkatalysatorschüttung 2 zonenweise zunehmende (ist besonders vorteilhaft) volumenspezifische Aktivität lässt sich für das erfindungsgemäße Verfahren somit in einfacher Weise z. B. dadurch einstellen, dass man die Schüttung in einer ersten Festbettkatalysatorschüttungszone mit einem hohen Anteil an inerten Verdünnungsformkörpern bezogen auf eine Sorte von Katalysatorformkörpern beginnt, und dann diesen Anteil an Verdünnungsformkörpern in Strömungsrichtung zonenweise verringert.

Eine solche erfindungsgemäß vorteilhafte zonenweise Zunahme der volumenspezifischen Aktivität ist aber auch z.B. dadurch möglich, dass man bei gleich bleibender Geometrie und Aktivmassenart eines Schalenkatalysatorformkörpers zonenweise die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse zonenweise den Anteil an Katalysatorformkörpern mit höherem Aktivmassengewichtsanteil steigert. Alternativ kann man auch die Aktivmassen selbst verdünnen, in dem man bei der Aktivmassenherstellung z. B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich z. B. auch dadurch erzielen, dass man bei Mischungen aus Vollkatalysatoren und aus Schalenkatalysatoren (bei identischer Aktivmasse) in entsprechender Weise das Mischungsverhältnis verändert. Des weiteren lässt sich eine Variation der volumenspezifischen Aktivität durch den Einsatz von Katalysatorgeometrien mit unterschiedlicher Schüttdichte erzielen (z. B. bei Vollkatalysatoren mit identischer Aktivmassenzusammensetzung der verschiedenen Geometrien). Selbstredend lassen sich die beschriebenen Varianten auch kombiniert anwenden.

Natürlich können für die Festbettkatalysatorschüttung 2 aber auch Mischungen aus Katalysatoren mit chemisch unterschiedlicher Aktivzusammensetzung und als Folge dieser verschiedenen Zusammensetzung unterschiedlicher Aktivität verwendet werden. Diese Mischungen können wiederum zonenweise in ihrer Zusammensetzung variiert und/ oder mit unterschiedlichen Mengen inerter Verdünnungsformkörper verdünnt werden.

Vorab und/oder im Anschluss an die Festbettkatalysatorschüttung 2 können sich ausschließlich aus Inertmaterial (z. B. nur Verdünnungsformkörpern) bestehende Schüttungen befinden (sie werden in dieser Schicht begrifflich nicht der Festbettkatalysatorschüttung 2 zugerechnet, da sie keine Formkörper enthalten, die Multimetalloxidaktivmasse aufweisen). Dabei können die für die Inertschüttung verwendeten Verdünnungsformkörper die gleich Geometrie wie die in der Festbettkatalysatorschüttung 2 verwendeten Verdünnungsformkörper aufweisen. Die Geometrie der für die Inertschüttung verwendeten Verdünnungsformkörper kann aber auch von der vorgenannten Geometrie der Katalysatorformkörper verschieden sein (z. B. kugelförmig anstatt ringförmig).

Häufig weisen die für solche Inertschüttungen verwendeten Formkörper die ringförmige Geometrie 7 mm x 7 mm x 4 mm bzw. 7 mm x 3 mm x 4 mm (jeweils Außendurchmesser x Länge x Innendurchmesser) oder die kugelförmige Geometrie mit dem Durchmesser d = 4 - 5 mm auf. Die Temperaturzonen A und B können sich beim erfindungsgemäßen Verfahren auch auf die Inertschüttungen erstrecken. Erfindungsgemäß vorteilhaft erfassen sowohl die Temperaturzone A als auch die Temperaturzone B für eine erfindungsgemäße Partialoxidation von Acrolein zu Acrylsäure jeweils nicht mehr als drei Festbettkatalysatorschüttungszonen, (erfindungsgemäß vorteilhaft wird wenigstens eine Festbettkatalysatorschüttungszone von beiden Temperaturzonen erfasst).

Erfindungsgemäß besonders bevorzugt umfasst die gesamte Festbettkatalysatorschüttung 2 nicht mehr als fünf, zweckmäßig nicht mehr als vier bzw. drei Festbettkatalysatorschüttungszonen.

Beim Übergang von einer Festbettkatalysatorschüttungszone in eine andere Festbettkatalysatorschüttungszone (in Strömungsrichtung des Reaktionsgasgemisches 2) sollte (bei einheitlicher Aktivmasse über die gesamte Festbettkatalysatorschüttung 2) die volumenspezifische Aktivmasse (d.h., das Gewicht der im Einheitsschüttungsvolumen enthaltenen Multimetalloxidaktivmasse) erfindungsgemäß zweckmäßig um wenigstens 5 Gew.-%, bevorzugt um wenigstens 10 Gew.-% zunehmen (dies gilt insbesondere auch bei einheitlichen Katalysatorformkörpern über die gesamte Festbettkatalysatorschüttung 2). In der Regel wird diese Zunahme bei einem erfindungsgemäßen Verfahren der heterogen katalysierten Partialoxidation von Acrolein zu Acrylsäure nicht mehr als 50 Gew.-%, meist nicht mehr als 40 Gew.-% betragen. Ferner sollte bei einheitlicher Aktivmasse über die gesamte Festbettkatalysatorschüttung 2 der Unterschied in der volumenspezifischen Aktivmasse derjenigen Festbettkatalysatorschüttungszone mit der geringsten volumenspezifischen Aktivität und derjenigen Festbettkatalysatorschüttungszone mit der höchsten volumenspezifischen Aktivität erfindungsgemäß vorteilhaft nicht mehr als 50 Gew.-%, vorzugsweise nicht mehr als 40 Gew.-% und besonders bevorzugt nicht mehr als 30 Gew.-% betragen.

Häufig wird bei einem erfindungsgemäßen Verfahren der heterogen katalysierten Partialoxidation von Acrolein zu Acrylsäure die Festbettkatalysatorschüttung 2 aus nur zwei Festbettkatalysatorschüttungszonen bestehen.

Erfindungsgemäß bevorzugt ist die in Strömungsrichtung des Reaktionsgasgemisches 2 letzte Festbettkatalysatorschüttungszone der Festbettkatalysatorschüttung 2 unverdünnt. D.h., sie besteht vorzugsweise ausschließlich aus Katalysatorformkörpern. Im Bedarfsfall kann sie auch aus einer Schüttung aus Katalysatorformkörpern bestehen, deren volumenspezifische Aktivität z. B. durch Verdünnung mit Inertmaterial abgesenkt, z. B. um 10 %, ist.

Besteht die Festbettkatalysatorschüttung 2 für eine heterogen katalysierte Partialoxidation von Acrolein zu Acrylsäure nur aus zwei Festbettkatalysatorschüttungszonen, ist es erfindungsgemäß in der Regel vorteilhaft, wenn die Festbettkatalysatorschüttungszone mit der höchsten volumenspezifischen Aktivität bis in die Temperaturzone A hineinragt (insbesondere dann, wenn in der Temperaturzone A und in der Temperaturzone B die Temperierung mittels eines fließenden Wärmeträgers erfolgt, der jeweils im Gegenstrom (über den Reaktor betrachtet) zum Reaktionsgasgemisch 2 strömt).

Besteht die Festbettkatalysatorschüttung 2 nur aus drei Festbettkatalysatorschüttungszonen, ist es erfindungsgemäß in der Regel gleichfalls vorteilhaft, wenn die Festbettkatalysatorschüttungszone mit der höchsten volumenspezifischen Aktivität bis in die Temperaturzone A hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch 2 strömt).

Besteht die Festbettkatalysatorschüttung 2 aus vier Festbettkatalysatorschüttungszonen, ist es erfindungsgemäß in der Regel vorteilhaft, wenn die Festbettkatalysatorschüttungszone mit der zweithöchsten volumenspezifischen Aktivität sowohl in die Temperaturzone A als auch in die Temperaturzone B hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch 2 strömt.

Im Fall einer Gleichstromführung von Reaktionsgasgemisch 2 und Wärmeträgern in den Temperaturzonen A und B kann es erfindungsgemäß vorteilhaft sein, wenn innerhalb der Festbettkatalysatorschüttung 2 die Festbettkatalysatorschüttungszone mit der höchsten volumenspezifischen Aktivität nicht in die Temperaturzone A hineinragt, sondern erst hinter dem Übergang von der Temperaturzone A in die Temperaturzone B ihren Anfang hat.

Die volumenspezifische Aktivität zwischen zwei Festbettkatalysatorschüttungszonen innerhalb der Festbettkatalysatorschüttung 2 lässt sich experimentell in einfacher Weise so differenzieren, dass unter identischen Randbedingungen (vorzugsweise den Bedingungen des ins Auge gefassten Verfahrens) über Festbettkatalysatorschüttungen der selben Länge, aber jeweils der Zusammensetzung der jeweiligen Festbettkatalysatorschüttungszone entsprechend, das gleich Acrolein enthaltende Reaktionsgasausgangsgemisch geführt wird. Die höhere umgesetzte Menge an Acrolein weist die höhere volumenspezifische Aktivität aus.

Beträgt die Gesamtlänge der Festbettkatalysatorschüttung 2 $L^2$, ist es erfindungsgemäß vorteilhaft, wenn sich im Bereich $X^2 \pm L^2 \dfrac{4}{100}$ bzw. im Bereich $X^2 \pm L^2 \dfrac{3}{100}$ bzw. im Bereich $X^2 \pm L^2 \dfrac{2}{100}$ kein Übergang von einer Festbettkatalysatorschüttungszone in eine andere Festbettkatalysatorschüttungszone befindet, wobei X der Ort innerhalb der Festbettkatalysatorschüttung 2 ist, an dem der Übergang von der Temperaturzone A in die Temperaturzone B erfolgt.

Erfindungsgemäß bevorzugt ist beim erfindungsgemäßen Verfahren die Festbettkatalysatorschüttung 2 in Strömungsrichtung des Reaktionsgasgemisches 2 wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h., z. B. auf einer Länge von 0,70 bis 1,50 m, bevorzugt 0,90 bis 1,20 m), jeweils der Gesamtlänge der Festbettkatalysatorschüttung 2, ein homogenes Gemisch oder zwei (mit abnehmender Verdünnung) aufeinander folgende homogene Gemische aus Katalysatorformkörpern und Verdünnungsformkörpern (wobei beide vorzugsweise im wesentlichen die gleich Geometrie aufweisen), wobei der Anteil der Verdünnungsformkörper so bemessen ist, dass die volumenspezifische Aktivmasse, bezogen auf eine nur aus den Katalysatorformkörpern bestehende Schüttung, um 10 bis 50 Gew.-%, vorzugsweise 20 bis 45 Gew.-% und besonders bevorzugt 25 bis 35 Gew.-% abgesenkt ist. Im Anschluss an diese erste bzw. an diese beiden ersten Zonen der Festbettkatalysatorschüttung 2 befindet sich dann erfindungsgemäß vorteilhaft bis zum Ende der Länge der Festbettkatalysatorschüttung 2 (d.h., z. B. auf einer Länge von 1,00 (bzw. 1,50) bis 3,50 m, bevorzugt 2,00 bis 3,00 m) entweder eine nur in geringerem Umfang (als in der ersten bzw. in den ersten beiden Zonen) verdünnte Schüttung der Katalysatorformkörper, oder, ganz besonders bevorzugt, eine alleinige Schüttung derselben Katalysatorformkörper, die auch in den ersten Zonen verwendet worden sind.

Das Vorgenannte trifft insbesondere dann zu, wenn in der Festbettkatalysatorschüttung 2 als Katalysatorformkörper Schalenkatalysatorringe oder Schalenkatalysatorkugeln eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 7 mm x 3 mm x 4 mm (Außendurchmesser x Länge x Innendurchmesser) auf.

Das oben Genannte trifft auch dann zu, wenn anstelle inerter Verdünnungsformkörper Schalenkatalysatorformkörper verwendet werden, deren Aktivmassenanteil um 2 bis 15 Gew.-% Punkte tiefer liegt, als der Aktivmassenanteil der Schalenkatalysatorformkörper am Ende der Festbettkatalysatorschüttung 2.

Eine reine Inertmaterialschüttung, deren Länge, bezogen auf die Länge der Festbettkatalysatorschüttung 2, zweckmäßig 5 bis 20 % beträgt, leitet in Strömungsrichtung des Reaktionsgasgemisches in der Regel die Festbettkatalysatorschüttung 2 ein. Sie dient normalerweise dem Zweck der Temperierung des Reaktionsgasgemisches 2.

Erfindungsgemäß vorteilhaft erstreckt sich nun bei den vorgenannten Festbettkatalysatorschüttungen 2 die Temperaturzone A (die sich erfindungsgemäß vorteilhaft auch auf die Vorschüttung aus Inertmaterial erstreckt) noch auf 5 bis 20 %, häufig auf 5 bis 15 % der Länge der in Strömungsrichtung des Reaktionsgasgemisches 2 letzten (volumenspezifisch aktivsten) Festbettkatalysatorschüttungszone der Festbettkatalysatorschüttung 2.

Selbstredend können sich beim erfindungsgemäßen Verfahren an die Temperaturzonen A, B weitere zusätzliche Temperaturzonen anschließen. Dies ist erfindungsgemäß jedoch nicht bevorzugt.

Für Schalenkatalysatoren (z. B. der Festbettkatalysatorschüttungen 1 bzw. 2) eignen sich insbesondere Trägerkörper, die eine erhöhte Oberflächenrauhigkeit aufweisen, da diese in der Regel eine erhöhte Haftfestigkeit der aufgebrachten Schale an Aktivmasse bedingt.

Vorzugsweise liegt die Oberflächenrauhigkeit $R_Z$ des Trägerkörpers im Bereich von 30 bis 200 $\mu$m, vorzugsweise 30 bis 100 $\mu$m (bestimmt gemäß DIN 4768 Blatt 1 mit einem "Hommel Tester" für DIN-ISO Oberflächenmessgrößen der Fa. Hommelwerke). Das Vorgenannte gilt insbesondere für Trägerkörper aus Steatit C 220 der Fa. CeramTec.. Grundsätzlich können die Trägermaterialien porös oder unporös sein.

In anwendungstechnisch zweckmäßiger Weise erfolgt die Durchführung eines erfindungsgemäßen Verfahrens der Partialoxidation von Propylen zu Acrolein in einem Zweizonenrohrbündelreaktor, wie er z. B. in den DE-A's 19910508, 19948523, 19910506 und 19948241 beschrieben ist. Eine bevorzugte Variante eines erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903218 offenbarten Zweizonenrohrbündelreaktoren sind für die Durchführung eines solchen Verfahrens geeignet.

D.h., in einfachster Weise befindet sich die für ein solches Verfahren erfindungsgemäß zu verwendende Festbettkatalysatorschüttung 1 (eventuell mit vor- und/oder nachangeordneten Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Temperaturzone. D.h., in einfachster Weise umströmt z. B. ein Salzbad A denjenigen Abschnitt der Rohre (die Temperaturzone A), in welchem sich die oxidative Umsetzung des Propens (beim einfachen Durchgang) bis zum Erreichen eines Umsatzes $U^A$ im erfindungsgemäß erforderlichen Bereich vollzieht und ein Salzbad B umströmt den Abschnitt der Rohre (die Temperaturzone B), in welchem sich die oxidative Anschlussumsetzung des Propens (beim einfachen Durchgang) bis zum Erreichen eines Umsatzwertes $U^B$ von wenigstens 90 mol-% vollzieht (bei Bedarf können sich an die erfindungsgemäß anzuwendenden Temperaturzonen A, B weitere Temperaturzonen anschließen, die auf individuellen Temperaturen gehalten werden).

Anwendungstechnisch zweckmäßig umfasst eine erfindungsgemäße Propylenpartialoxidation zu Acrolein keine solchen weiteren Temperaturzonen. D.h., das Salzbad B umströmt zweckmäßig den Abschnitt der Rohre, in welchem sich die oxidative Anschlussumsetzung des Propens (beim einfachen Durchgang) bis zu einem Umsatzwert $\geq$ 90 mol-%, oder $\geq$ 92 mol-% oder $\geq$ 94 mol-% oder mehr vollzieht.

Die beiden Salzbäder A, B können erfindungsgemäß relativ zur Strömungsrichtung des durch die Reaktionsrohre strömenden Reaktionsgasgemisches 1 im Gleichstrom oder im Gegenstrom durch den die Reaktionsrohre umgebenden Raum geführt werden. Selbstverständlich kann erfindungsgemäß auch in der Temperaturzone A eine Gleichströmung und in der Temperaturzone B eine Gegenströmung (oder umgekehrt) angewandt werden.

Selbstverständlich kann man in allen vorgenannten Fallkonstellationen innerhalb der jeweiligen Temperaturzone der, relativ zu den Reaktionsrohren, erfolgenden Parallelströmung der Salzschmelze noch eine Querströmung überlagern, so dass die einzelne Reaktionszone einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Zweckmäßigerweise wird ein Reaktionsgaseingangsgemisch 1 der Festbettkatalysatorschüttung 1 auf die Temperaturzone A vorerwärmt zugeführt.

Üblicherweise sind in den Zweizonenrohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 2 bis 4 m, bevorzugt 2,5 bis 3,5 m. In jeder Temperaturzone belegt die Festbettkatalysatorschüttung 1 wenigstens 60 % bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000 oder bis 40000. Rohrbündelreaktoren mit einer oberhalb von 50000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z. B. EP-B 468290).

Als Wärmeaustauschmittel eignen sich auch für die Zweizonenfahrweise insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren die Fließgeschwindigkeit innerhalb der beiden erforderlichen Wärmeaustauschmittelkreisläufen so gewählt, dass die Temperatur des Wärmeaustauschmittels von der Eintrittsstelle in die **T**emperaturzone bis zur Austrittstelle aus der **T**emperaturzone (bedingt durch die Exothermie der Reaktion) um 0 bis 15 °C ansteigt. D.h., das vorgenannte ΔT kann erfindungsgemäß 1 bis 10 °C, oder 2 bis 8 °C oder 3 bis 6 °C betragen.

Die Eintrittstemperaturen der Wärmeaustauschmittel in die Temperaturzonen A, B der Zweizonenrohrbündelreaktoren sind dabei bei einer Propylenpartialoxidation zu Acrolein erfindungsgemäß so zu wählen, dass sie den für diese Reaktion in dieser Schrift für die Temperaturzonen A, B geforderten Temperaturen sowie Temperaturunterschieden $\Delta T^{BA}$ entsprechen. Im erfindungsgemäßen Langzeitbetrieb sind sie erfindungsgemäß zu verändern.

Es sei an dieser Stelle auch noch einmal darauf hingewiesen, dass für die Durchführung einer erfindungsgemäßen Propylenpartialoxidation zu Acrolein insbesondere auch der in der DE-AS 2201528 beschriebene Zweizonenrohrbündelreaktortyp verwendet werden kann, der die Möglichkeit beinhaltet, vom heißen Wärmeaustauschmittel der Temperaturzone B eine Teilmenge an die Temperaturzone A abzuführen, um gegebenenfalls ein Anwärmen eines kalten Reaktionsgaseingangsgemisches 1 oder eines kalten Kreisgases zu bewirken. Ferner kann die Rohrbündelcharakteristik innerhalb einer individuellen Temperaturzone wie in der EP-A 382098 beschrieben gestaltet werden.

Im Fall einer zweistufigen heterogen katalysierten Partialoxidation von Propylen zu Acrylsäure wird man erfindungsgemäß zweckmäßig zwei erfindungsgemäße Zweizonenverfahren hintereinanderschalten. Eine erfindungsgemäße Partialoxidation von Propylen zu Acrolein bildet dabei die erste Reaktionsstufe und eine erfindungsgemäße Partialoxidation von Acrolein zu Acrylsäure bildet dabei die hinter die erste Reaktionsstufe geschaltete zweite Reaktionsstufe.

Dabei ist es zweckmäßig, das die erste Reaktionsstufe verlassende Produktgasgemisch vor dem Eintritt in die zweite Reaktionsstufe auf direkte und/oder indirekte Weise abzukühlen, um so eine Nachvollverbrennung von Teilen des in der ersten Reaktionsstufe gebildeten Acroleins zu unterdrücken. Üblicherweise wird dazu zwischen die beiden Reaktionsstufen ein Nachkühler geschaltet. Dies kann im einfachsten Fall ein indirekter Rohrbündelwärmeübertäger sein. Das Produktgasgemisch wird dabei in der Regel durch die Rohre geführt und um die Rohre wird ein Wärmetauschermedium geführt, dessen Art der für die Rohrbündelreaktoren empfohlenen Wärmetauschermedien entsprechen kann. Mit Vorteil ist das Rohrinnere mit inerten Füllkörpern (z. B. Spiralen aus Edelstahl, Ringe aus Steatit, Kugeln aus Steatit etc.) gefüllt. Selbige verbessern den Wärmeaustausch und fangen gegebenenfalls aus der Festbettkatalysatorschüttung der ersten Reaktionsstufe sublimierendes Molybdäntrioxid vor einem Eintritt desselben in die zweite Reaktionsstufe ab. Es ist von Vorteil, wenn der Nachkühler aus mit Zinksilicatfarbe beschichtetem rostfreiem Stahl gefertigt ist.

Als Quelle für den in der ersten Reaktionsstufe erforderlichen molekularen Sauerstoff kommt sowohl Luft, als auch an molekularem Stickstoff entreicherte Luft (z. B. ≥ 90 Vol.-% $O_2$, ≤ 10 Vol.-% $N_2$) in Betracht.

Anwendungstechnisch zweckmäßig wird das Produktgasgemisch der ersten Reaktionsstufe im bereits erwähnten Nachkühler auf eine Temperatur von 210 bis 290 °C, häufig 230 bis 280 °C oder 250 bis 270 °C abgekühlt. Dabei kann die Abkühlung des Produktgasgemisches der ersten Reaktionsstufe durchaus auf Temperaturen erfolgen, die unterhalb der Temperatur der Temperaturzone A zweiten Reaktionsstufe liegen. Die beschriebene Nachkühlung ist jedoch keineswegs zwingend und kann insbesondere dann in aller Regel entfallen, wenn der Weg des Produktgasgemisches von der ersten Reaktionsstufe in die zweite Reaktionsstufe kurz gehalten wird. Üblicherweise wird ein solches zweistufiges erfindungsgemäßes Verfahren ferner so verwirklicht, dass man den Sauerstoffbedarf in der zweiten Reaktionsstufe nicht bereits durch einen entsprechend hohen Sauerstoffgehalt des Reaktionsgaseingangsgemisches 1 deckt, sondern den benötigten Sauerstoff im Bereich zwischen erster und zweiter Reaktionsstufe zugibt ("Sekundärgaszusatz"). Dies kann vor, während, nach und/oder zur Nachkühlung erfolgen. Als Quelle für den in der zweiten Reaktionsstufe erforderlichen molekularen Sauerstoff kommen sowohl reiner Sauerstoff als auch Gemische aus Sauerstoff und Inertgas, z. B. Luft oder an molekularem Stickstoff entreicherte Luft (z. B. ≥ 90 Vol-% $O_2$, ≤ 10 Vol-% $N_2$) in Betracht. Die Zugabe der Sauerstoffquelle erfolgt regelmäßig in auf den Reaktionsdruck komprimierter Form. Selbstredend kann bei einem solchen zweistufigen Verfahren der Sauerstoffbedarf in der zweiten Reaktionsstufe bereits durch einen entsprechend hohen Sauerstoffbedarf in der ersten Reaktionsstufe gedeckt werden. Natürlich kann bei Bedarf als Sekundärgas auch ein inertes Verdünnungsgas zugesetzt werden.

Wie die Durchführung der ersten Reaktionsstufe erfolgt auch die Durchführung der zweiten Reaktionsstufe eines solchen zweistufigen Verfahrens in anwendungstechnisch zweckmäßiger Weise in einem Zweizonenrohrbündelreaktor, wie er für die erste Reaktionsstufe bereits beschrieben wurde. Die Ausführungen hinsichtlich des Zweizonenrohrbündelreaktors für die erste Reaktionsstufe gelten deshalb auch für den Zweizonenrohrbündelreaktor für die zweite Reaktionsstufe (dies gilt auch dann, wenn "eine zweite Reaktionsstufe" unabhängig von einer vorgeschalteten ersten Reaktionsstufe durchgeführt wird.

D.h., in einfacher Weise befindet sich auch die für eine zweite Reaktionsstufe bzw. allgemein für eine erfindungsgemäß heterogen katalysierte Partialoxidation von Acrolein zu Acrylsäure zu verwendende Festbettkatalysatorschüttung 2 (gegebenenfalls einschließlich der Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Temperaturzone.

D.h., in einfacher Weise umströmt z. B. ein Salzbad A diejenigen Abschnitte der Rohre (die Temperaturzone A), in welchem sich die oxidative Umsetzung des Acroleins (beim einfachen Durchgang) bis zum Erreichen eines $U^A$ im erfindungsgemäß erforderlichen Bereich vollzieht und ein Salzbad B umströmt den Abschnitt der Rohre (die Temperaturzone B), in welchem sich die oxidative Anschlussumsetzung des Acroleins (beim einfachen Durchgang) bis zum Erreichen eines Umsatzwertes von wenigstens 90 mol-% vollzieht (bei Bedarf können sich an die erfindungsgemäß anzuwendenden Temperaturzonen A, B weitere Temperaturzonen anschließen, die auf individuellen Temperaturen gehalten werden).

Anwendungstechnisch zweckmäßig umfasst auch eine erfindungsgemäße Acroleinpartialoxidation keine solchen weiteren Temperaturzonen. D.h., das Salzbad B umströmt zweckmäßig den Abschnitt der Rohre, in welchem sich die oxidative (Anschluss) Umsetzung des Acroleins (beim einfachen Durchgang) bis zu einem Umsatzwert von $\geq$ 92 mol-%, oder $\geq$ 94 mol-% oder $\geq$ 96 mol-% oder $\geq$ 98 mol-% und häufig sogar $\geq$ 99 mol-% oder mehr vollzieht.

Die beiden Salzbäder A, B können erfindungsgemäß relativ zur Strömungsrichtung des durch die Reaktionsrohre strömenden Reaktiongasgemisches 2 im Gleichstrom oder im Gegenstrom durch den die Reaktionsrohre umgebenden Raum geführt werden. Selbstverständlich kann erfindungsgemäß auch in der Temperaturzone A eine Gleichströmung und in der Temperaturzone B eine Gegenströmung (oder umgekehrt) angewandt werden.

Selbstredend kann man in allen vorgenannten Fallkonstellationen innerhalb der jeweiligen Temperaturzone der, relativ zu den Reaktionsrohren, erfolgenden Parallelströmung der Salzschmelze noch eine Querströmung überlagern, so dass die einzelne Reaktionszone einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Zweckmäßigerweise wird auch ein Reaktionsgaseingangsgemisch 2 der Festbettkatalysatorschüttung 2 auf die Temperatur der Zone A vorerwärmt zugeführt.

Üblicherweise sind in den vorgenannten Zweizonen-Rohrbündelreaktoren für die zweite Reaktionsstufe die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 3 bis 4, bevorzugt 3,5 m. In jeder Temperaturzone belegt die Festbettkatalysatorschüttung 2 wenigstens 60 %, bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000 oder bis 40000. Rohrbündelreaktoren mit einer oberhalb von 50000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren der zweiten Reaktionsstufe die Fließgeschwindigkeit innerhalb der beiden erforderlichen Wärmeaustauschmittelkreisläufe so gewählt, dass die Temperatur des Wärmeaustauschmittels von der Eintrittstelle in die Temperaturzone bis zur Austrittsstelle aus der Temperaturzone um 0 bis 15 °C ansteigt. D.h., das vorgenannte $\Delta T$ kann erfindungsgemäß 1 bis 10 °C, oder 2 bis 8 °C oder 3 bis 6 °C betragen.

Die Eintrittstemperaturen der Wärmeaustauschmittel in die Temperaturzonen A, B der Zweizonenrohrbündelreaktoren sind dabei bei einer Acroleinpartialoxidation zu Acrylsäure erfindungsgemäß so zu wählen, dass sie den für diese Reaktion in dieser Schrift für die Temperaturzonen A, B geforderten Temperaturen und Temperaturunterschieden $\Delta T^{BA}$ entsprechen. Im erfindungsgemäßen Langzeitbetrieb sind sie erfindungsgemäß zu verändern.

Es sei an dieser Stelle auch noch einmal darauf hingewiesen, dass für eine Durchführung einer erfindungsgemäßen Partialoxidation von Acrolein zu Acrylsäure insbesondere auch der in der DE-AS 2201528 beschriebene Zweizonenrohrbündelreaktortyp verwendet werden kann, der die Möglichkeit beinhaltet, vom heißen Wärmeaustauschmittel der Reaktionszone B eine Teilmenge an die Reaktionszone A abzuführen, um gegebenenfalls ein Anwärmen eines zu kalten Reaktionsgaseingangsgemisches 2 oder eines kalten Kreisgases zu bewirken. Ferner kann die Rohrbündelcharakteristik, innerhalb einer individuellen Reaktionszone wie in der EP-A 382 098 beschrieben gestaltet werden.

Selbstredend können zur Durchführung einer erfindungsgemäßen zweistufigen Partialoxidation von Propylen zu Acrylsäure zwei Zweizonenrohrbündelreaktoren zu einem Vierzonenrohrbündelreaktor verschmolzen werden, wie es in der WO 01/36364 beschrieben ist. Normalerweise befindet sich in diesen Fällen zwischen der Festbettkatalysatorschüttung 1 und der Festbettkatalysatorschüttung 2 eine Inertschüttung. Allerdings kann auf eine solche Zwischeninertschüttung auch verzichtet werden. Die Länge der Reaktionsrohre entspricht im Verschmelzungsfall vielfach der Summe der Längen der nicht verschmolzenen Rohrbündelreaktoren.

Generell ist es günstig, eine erfindungsgemäße Partialoxidation von Propylen zu Acrolein so zu betreiben, dass der Propylengehalt im Produktgasgemisch dieser Partialoxidation den Wert 10000 Gew.-ppm, vorzugsweise 6000 Gew.-ppm und besonders bevorzugt 4000 bis 2000 Gew.-ppm nicht übersteigt.

Generell ist es günstig, eine erfindungsgemäße Partialoxidation von Acrolein zu Acrylsäure so zu betreiben, dass der Acroleingehalt im Produktgasgemisch dieser Partialoxidation den Wert 1500 Gew.-ppm, vorzugsweise 600 Gew.-ppm und besonders bevorzugt 350 Gew.-ppm nicht übersteigt.

Der Propenanteil im Reaktionsgasausgangsgemisch 1 kann beim erfindungsgemäßen Verfahren z. B. bei Werten von 3 bis 25 Vol.-%, oft 4 bis 20 Vol.-%, oder 5 bis 15 Vol.-%, häufig bei 6 bis 12 Vol.-% bzw. 6 bis 8 Vol.-% liegen (jeweils bezogen auf das Gesamtvolumen). Als Propylenquelle eignen sich insbesondere "polymer grade Propylen" und "chemical grade Propylen" gemäß WO 2004/009525.

Häufig wird man das erfindungsgemäße Propylen -> Acrolein Verfahren bei einem Propen : Sauerstoff : indifferente Gase (einschließlich Wasserdampf) Volumenverhältnis im Reaktionsgasausgangsgemisch 1 von 1 : (1,0 bis 3,0) : (5 bis 25), vorzugsweise 1 : (1,7 bis 2,3) : (10 bis 15) durchführen. Im Regelfall wird das indifferente (inerte) Gas zu wenigstens 20 % seines Volumens aus molekularem Stickstoff bestehen. Es kann aber auch zu ≥ 30 Vol.-%, oder zu ≥ 40 Vol.-%, oder zu ≥ 50 Vol.-%, oder zu ≥ 60 Vol.-%, oder zu ≥ 70 Vol.-%, oder zur ≥ 80 Vol.-%, oder zu ≥ 90 Vol.-%, oder zu ≥ 95 Vol.-% aus molekularem Stickstoff bestehen (mögliche Inertgase sind neben molekularem Stickstoff z.B. Gase wie Propan, Ethan, Methan, Pentan, Butan, $CO_2$ CO, Wasserdampf und/oder Edelgase). Natürlich kann das inerte Verdünnungsgas bei einer erfindungsgemäßen Propylenpartialoxidation zu Acrolein auch zu bis zu 50 mol-%, bzw. bis zu 75 mol-% und mehr aus Propan bestehen. Bestandteil des Verdünnungsgases kann auch Kreisgas sein, wie es bei der zweistufigen Propylenpartialoxidation zu Acrylsäure nach der Abtrennung der Acrylsäure aus dem Produktgasgemisch verbleibt.

Vorgenannte Zusammensetzungsbereiche gelten auch für solche zweistufigen Verfahren, und zwar sowohl in Fällen von Sekundärgaszufuhr als auch in Fällen wo kein Sekundärgas zugeführt wird.

Erfindungsgemäß geeignete Reaktionsgasausgangsgemische 1 sind z. B. solche, die aus

| | |
|---|---|
| 6 bis 15 (bevorzugt 7 bis 11) Vol-% | Propen, |
| 4 bis 20 (bevorzugt 6 bis 12) Vol-% | Wasser, |
| ≥ 0 bis 10 (bevorzugt ≥ 0 bis 5) Vol-% | von Propen, Wasser Sauerstoff und Stickstoff verschiedenen Bestandteilen, |

soviel molekularem Sauerstoff, dass das molare Verhältnis von enthaltenem molekularem Sauerstoff zu enthaltenem Propen 1,5 bis 2,5 (bevorzugt 1,6 bis 2,2) beträgt, und als Restmenge bis zu 100 Vol.-% Gesamtmenge aus molekularem Stickstoff zusammengesetzt sind,

wie sie die DE-A 10302715 empfiehlt.

Insbesondere bei hohen Propen- bzw. Acroleinbelastungen der jeweiligen Festbettkatalysatorschüttung wird die Mitverwendung von inerten Verdünnungsgasen mit hoher spezifischer Wärme empfohlen.

Der Acroleinanteil im Reaktionsgasausgangsgemisch 2 kann erfindungsgemäß z. B. bei Werten von 3 bis 25 Vol.-%, oft 4 bis 20 Vol.-%, oder 5 bis 15 Vol.-%, häufig bei 4 bis 10 Vol.-% bzw. 5 bis 8 Vol.-% liegen (jeweils bezogen auf das Gesamtvolumen).

Häufig wird man das erfindungsgemäße Verfahren mit einem im Reaktionsgaseingangsgemisch 2 vorliegenden Acrolein : Sauerstoff: Wasserdampf : Inertgas - Volumenverhältnis (NI) von 1 : (1 bis 3) : (0 bis 20) : (3 bis 30), vorzugsweise von 1 : (1 bis 3) : (0,5 bis 10) : (7 bis 10) ausführen.

Selbstredend kann man das erfindungsgemäße Verfahren aber auch mit einem im Reaktionsgaseingangsgemisch 2 vorliegenden Acrolein : Sauerstoff : Wasserdampf : Sonstige - Volumenverhältnis (NI) von 1 : (0,9 bis 1,3) : (2,5 bis 3,5) : (10 bis 12) ausführen.

An dieser Stelle sei noch festgehalten, dass als Aktivmassen sowohl für die Festbettkatalysatorschüttung 1 als auch für die Festbettkatalysatorschüttung 2 auch die Multimetalloxidmassen der DE-A 10261186 günstig sind.

Insbesondere im Fall einer zweistufigen erfindungsgemäßen heterogen katalysierten Gas-Phasen-Partialoxidation von Propylen zu Acrylsäure kommen auch die nachfolgenden Beschaffenheiten des Reaktionsgaseingangsgemisch in Betracht. Beispielsweise kann das Reaktionsgaseingangsgemisch 1 ≥ 0,01, oder ≥ 0,1, oder ≥ 0,5, oder ≥ 2 Vol.-% an $CO_2$ enthalten. Meist wird der vorgenannten $CO_2$-Gehalt ≤ 25 Vol.-% betragen.

Insbesondere dann, wenn als Quelle für den molekularen Sauerstoff beim erfindungsgemäßen Verfahren Luft verwendet wird, wird das Reaktionsgaseingangsgemisch 1 als weiteres inertes Verdünnungsgas molekularen Stickstoff enthalten. Grundsätzlich kann das Reaktionsgaseingangsgemisch 1 beim erfindungsgemäßen Verfahren ≥ 1 Vol.-%, oder ≥ 5 Vol.-%, oder ≥ 10 Vol.-%, oder ≥ 20 Vol.-%, oder ≥ 30 Vol.-%, oder ≥ 40 Vol.-% an molekularem Stickstoff enthalten. In der Regel wird der Gehalt des Reaktionsgaseingangsgemischs 1 an molekularem Stickstoff jedoch bei Werten ≤ 80 mol-%, oder ≤ 70 mol-%, oder ≤ 60 mol-% liegen.

Auch kann das Reaktionsgaseingangsgemisch 1 (wie bereits gesagt) Propan als inertes Verdünnungsgas enthalten. Dieser Propangehalt des Reaktionsgaseingangsgemischs 1 kann bis zu 70 Vol.-% (z. B. 5 bis 70 Vol.-%), bzw. bis zu 60 Vol.-%, oder bis 50 Vol.-%, oder bis zu 40 Vol.-%, oder bis 30 Vol.-%, oder bis 20 Vol.-%, oder bis zu 10 Vol.-% betragen. Häufig liegt dieser Propangehalt bei $\geq 0,5$ bzw. $\geq 1$ Vol.-%. Er kann aber auch bei Werten von $\geq 0,01$ Vol.-%, oder $\geq 0,02$ Vol.-%, oder $\geq 0,03$ Vol.-% liegen. In der Regel enthält das Reaktionsgaseingangsgemisch 1 $\leq 10$ Vol.-%, vielfach $\leq 5$ Vol.-% an Propan.

Dieses Propan kann beim erfindungsgemäßen Verfahren z. B. bewusst als dem Reaktionsgaseingangsgemisch 1 separat zuzuführendes inertes Verdünnungsgas zugesetzt werden.

Selbstverständlich kann das Propan aber auch dadurch Bestandteil das Reaktionsgaseingangsgemisch 1 werden, dass als Propylenquelle für selbiges eine partielle Dehydrierung oder Oxidehydrierung von Propan fungiert (in der Regel werden diese heterogen katalysiert erfolgen). D. h., das im Reaktionsgaseingangsgemisch 1 enthaltene Propylen kann dem Reaktionsgaseingangsgemisch 1 wenigstens teilweise unter Begleitung von nicht umgesetztem Propan aus einer partiellen Dehydrierung (z. B. homogen und/oder heterogen katalysiert, im Beisein und/oder unter Ausschluss von molekularem Sauerstoff) zugeführt werden.

Das erfindungsgemäße Verfahren umfasst insbesondere auch solche Ausführungsformen, bei denen das Reaktionsgaseingangsgemisch 1 > 0 bis 35 Vol.-%, häufig 1 bis 25 Vol.-%, oder 5 bis 15 Vol.-%, bzw. bis 10 Vol.-% $H_2O$ enthält.

Typische Reaktionsgaseingangsgemische 1 sind z. B. solche, die enthalten:

| | |
|---|---|
| 5 oder 6 bis 11 Vol.-% | Propen, |
| 2 oder 6 bis 12 Vol.-% | Wasser, |
| > 0, häufig $\geq 0,5$ oder $\geq 1$ bis 10 Vol.-% | Propan, |
| $\geq 0$ bis 5 Vol.-% | von Propen, Propan, Wasser, Sauerstoff und Stickstoff verschiedene Bestandteile, |

**soviel molekularen Sauerstoff, dass $V_1$ 1 bis 3 beträgt, und** als Restmenge bis zu 100 Vol.-% Gesamtmenge molekularen Stickstoff.

Erfindungsgemäße Reaktionsgaseingangsgemische 1 können auch enthalten:

| | |
|---|---|
| 6 bis 9 Vol.-% | Propylen, |
| 8 bis 18 Vol.-% | molekularer Sauerstoff, |
| 6 bis 30 bzw. bis 35 Vol.-% | Propan und |
| 32 bis 72 Vol.-% | molekularer Stickstoff. |

Erfindungsgemäße Reaktionsgaseingangsgemische 2 können z. B. enthalten:

| | |
|---|---|
| 4,5 bis 8 Vol.-% | Acrolein, |
| 2,25 bis 9 Vol.-% | molekularer Sauerstoff, |
| 6 bis 30 bzw. bis 35 Vol.-% | Propan, |
| 32 bis 72 Vol.-% | molekularer Stickstoff, |
| 5 bis 30 Vol.-% | Wasserdampf. |

Erfindungsgemäße Reaktionsgaseingangsgemische 1 können aber auch bis zu 20 Vol.-% $H_2$ enthalten.

D.h., Reaktionsgaseingangsgemische 1 des erfindungsgemäßen Verfahrens können auch enthalten:

| | |
|---|---|
| 4 bis 25 Vol.-% | Propylen, |
| 6 bis 70 Vol.-% | Propan, |
| 5 bis 60 Vol.-% | $H_2O$, |
| 8 bis 65 Vol.-% | $O_2$ und |
| 0,3 bis 20 Vol.-% | $H_2$. |

Das erfindungsgemäße Verfahren ist aber auch dann günstig, wenn das Reaktionsgaseingangsgemisch 1 0,1 bis 30 Vol.-% $CO_2$ enthält.

Erfindungsgemäß mögliche Reaktionsgaseingangsgemische 2 können auch enthalten:

| | |
|---|---|
| 3 bis 25 Vol.-% | Acrolein, |
| 5 bis 65 Vol.-% | molekularer Sauerstoff, |
| 6 bis 70 Vol.-% | Propan, |
| 0,3 bis 20 Vol.-% | molekularer Wasserstoff und |
| 8 bis 65 Vol.-% | Wasserdampf. |

Erfindungswesentlich ist, dass für alle vorgenannten Konstellationen das erfindungsgemäße Verfahren jeweils für beide Stufen sowohl dann angewendet werden kann, wenn die beiden Stufen voneinander unabhängig betrieben werden, als auch wenn sie wie vorstehend dargestellt in Hintereinanderschaltung betrieben werden.

An dieser Stelle sei nochmals erwähnt, dass insbesondere ein Teil des Reaktionsgaseingangsgemischs 1 sogenanntes Kreisgas sein kann. Hierbei handelt es sich um Gas, das z. B. bei einer erfindungsgemäßen zweistufigen Partialoxidation von Propylen zu Acrylsäure nach der Produktabtrennung (Acrylsäureabtrennung) vom Produktgasgemisch der zweiten Stufe verbleibt und bei einer Hintereinanderschaltung der beiden Stufen in der Regel zum Teil als inertes Verdünnungsgas zum Beschicken der ersten und/oder zweiten Stufe rückgeführt wird.

Eine typische Kreisgaszusammensetzung lautet:

| | |
|---|---|
| 0 - 0,1 Vol.-% | sonstige, z. B. Diphenyl, Diphenylether und/oder Dimethylphthalat, |
| 0 - 0,1 Vol.-% | Acrylsäure, |
| 0 - 0,1 Vol.-% | Acrolein, |
| 3 - 5 Vol.-% | Sauerstoff, |
| 1 - 5 Vol.-% | Wasserdampf, |
| 0 - 3 Vol.-% | Kohlenmonoxid, |
| 0 - 8 Vol.-% | Kohlendioxid, |
| 0 - 2 Vol.-% | Propan, |
| 0,1 - 0,5 Vol.-% | Propylen, |
| 85 - 95 Vol.-% | Stickstoff. |

Dabei kann die Acrylsäureabtrennung z. B. wie in der EP-A 982 287, der EP-A 982 289, der DE-A 199 24 532, der DE-A 101 15 277, der DE-A 196 06 877, der DE-A 197 40 252, der DE-A 196 27 847, der DE-A 100 53 086, der EP-A 982 288 und der DE-A 196 27 847 beschrieben abgetrennt werden.

Erfindungsgemäß günstige Ausgestaltungen eines Zweizonenrohrbündelreaktors für eine erfindungsgemäße heterogen katalysierte partielle Gasphasenoxidation von Propylen zu Acrolein (z. B. als erste Reaktionsstufe eines zweistufigen Acrylsäureherstellungsverfahrens) können wie folgt beschaffen sein (die konstruktive Detailgestaltung kann wie in den Gebrauchsmusteranmeldungen 202 19 277.6, 2002 19 278.4 und 202 19 279.2 bzw. in den PCT-Anmeldungen PCT/EP02/14187, PCT/EP02/14188 oder PCT/EP02/14189 erfolgen):

Kontaktrohre:

| | |
|---|---|
| Material der Kontaktrohre: | ferritischer Stahl; |
| Abmessungen der Kontaktrohre: | z.B. 3500 mm Länge; |
| | z. B. 30 mm Außendurchmesser; |
| | z. B. 2 mm Wandstärke; |

Anzahl der Kontaktrohre im Rohrbündel: z.B. 30000, oder 28000, oder 32000, oder 34000, oder 36000, oder 40000; zusätzlich bis zu 10 Thermorohre (wie in EP-A 873 783 und EP-A 12 70 065 beschrieben), die wie die Kontaktrohre beschickt sind (schneckenartig von ganz außen nach innen drehend) z. B. der selben Länge und Wandstärke, aber mit einem Außendurchmesser von z. B. 33,4 mm und einer zentrierten Thermohülse von z. B. 8 mm Außendurchmesser und z. B. 1 mm Wandstärke;

Reaktor (Material wie die Kontaktrohre):

Zylinderförmiger Behälter eines Innendurchmessers von 6000 - 8000 bzw. bis 10000 mm;

Reaktorhauben plattiert mit Edelstahl des Typs 1,4541; Plattierungsdicke: einige mm;

ringförmig angeordnetes Rohrbündel, z. B. mit einem freien zentralen Raum;

Durchmesser des zentralen freien Raumes: z.B. 1000 - 2500 mm (z. B. 1200 mm, oder 1400 mm, oder 1600 mm, oder 1800 mm, oder 2000 mm, oder 2200 mm, oder 2400 mm);

normalerweise homogene Kontaktrohrverteilung im Rohrbündel (6 äquidistante Nachbarrohre pro Kontaktrohr), Anordnung in gleichseitigem Dreieck, Kontaktrohrteilung (Abstand der zentrischen Innenachsen von zueinander nächstliegenden Kontaktrohren): 35 - 45 mm, z. B. 36 mm, oder 38 mm, oder 40 mm, oder 42 mm, oder 44 mm;

die Kontaktrohre sind mit ihren Enden in Kontaktrohrböden (oberer Boden und unterer Boden z. B. mit einer Dicke von 100 - 200 mm) abdichtend befestigt und münden am oberen Ende in eine mit dem Behälter verbundene Haube, die einen Zulass für das Reaktionsgaseingangsgemisch 1 aufweist; ein z.B. auf der halben Kontaktrohrlänge befindliches Trennblech einer Dicke von 20 - 100 mm, teilt den Reaktorraum symmetrisch in zwei Temperaturzonen A (obere Zone) und B (untere Zone); jede Temperaturzone wird durch eine Umlenkscheibe in 2 äquidistante Längsabschnitte geteilt;

die Umlenkscheibe weist bevorzugt Ringgeometrie auf; die Kontaktrohre sind mit Vorteil am Trennblech abdichtend befestigt; an den Umlenkscheiben sind sie nicht abdichtend befestigt, so dass die Querströmungsgeschwindigkeit der Salzschmelze innerhalb einer Zone möglichst konstant ist;

jede Zone wird durch eine eigene Salzpumpe mit Salzschmelze als Wärmeträger versorgt; die Zufuhr der Salzschmelze ist z. B. unterhalb der Umlenkscheibe und die Entnahme ist z. B. oberhalb der Umlenkscheibe;

aus beiden Salzschmelzekreisen wird z. B. ein Teilstrom entnommen und z. B. in einem gemeinsamen oder zwei getrennten indirekten Wärmetauschern abgekühlt (Dampferzeugung);

im ersten Fall wird der abgekühlte Salzschmelzestrom aufgeteilt, mit dem jeweiligen Reststrom vereinigt und durch die jeweilige Pumpe in den entsprechenden Ringkanal, der die Salzschmelze über den Behälterumfang verteilt, in den Reaktor gedrückt;

durch im Reaktormantel befindliche Fenster gelangt die Salzschmelze zum Rohrbündel; die Einströmung erfolgt z. B. in radialer Richtung zum Rohrbündel;

die Salzschmelze fließt in jeder Zone der Vorgabe des Umlenkblechs folgend z. B. in der Abfolge

- von außen nach innen,
- von innen nach außen,

um die Kontaktrohre;

durch um den Behälterumfang angebrachte Fenster sammelt sich die Salzschmelze an jedem Zonenende in einem um den Reaktormantel angebrachten Ringkanal, um einschließlich Teilstromkühlung im Kreis gepumpt zu werden;

über jede Temperaturzone wird die Salzschmelze von unten nach oben geführt;

Das Reaktionsgasgemisch verlässt den Reaktor der ersten Stufe mit einer Temperatur wenige Grad höher als die entsprechende Salzbadeintrittstemperatur des ersten Reaktors. Das Reaktionsgasgemisch wird für die Weiterverarbeitung zweckmäßigerweise in einem separaten Nachkühler, der dem Reaktor der 1. Stufe nachgeschaltet ist, auf 220 °C bis 280 °C, bevorzugt 240 °C bis 260 °C abgekühlt.

Der Nachkühler ist in der Regel unterhalb des unteren Rohrbodens angeflanscht und besteht normalerweise aus Rohren mit ferritischem Stahl. In die Rohre des Nachkühlers sind mit Vorteil innen Edelstahl-Blechspiralen, die teiloder vollgewendelt sein können, zur Verbesserung des Wärmeübergangs eingeführt.

Salzschmelze:

Als Salzschmelze kann ein Gemisch aus 53 Gew.-% Kaliumnitrat, 40 Gew.-% Natriumnitrit und 7 Gew.-% Natriumnitrat verwendet werden; beide Reaktionszonen und der Nachkühler wenden mit Vorteil eine Salzschmelze derselben Zusammensetzung an; die in den Reaktionszonen umgepumpte Salzmenge kann je Zone ca. 10000 $m^3$/h betragen. Die Salzschmelzen der beiden Temperaturzonen können jeweils durch entsprechende Schmelzentnahme in einem getrennten Salzbadkühler oder auch in einem gemeinsamen Salzbadkühler gekühlt werden.

Stromführung:

das Reaktionsgaseingangsgemisch 1 strömt zweckmäßig von oben nach unten durch den Erststufenreaktor, während die unterschiedlich temperierten Salzschmelzen der einzelnen Zonen zweckmäßig von unten nach oben gefördert werden;

Kontaktrohr- und Thermorohrbeschickung (von oben nach unten) z. B.:

Abschnitt 1:  50 cm Länge
Steatitringe der Geometrie 7 mm x 7 mm x 4 mm (Außendurchmesser x Länge x Innendurchmesser) als Vorschüttung. Alternativ können hier als Vorschüttung auch Steatitringe der Geometrie 7 mm x 3 mm x 4 mm verwendet werden.

Abschnitt 2:  140 cm Länge
Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt 3.

Abschnitt 3:  160 cm Länge
Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: $[Bi_2W_2O_9$ x 2 $WO_3]_{0,5}$ $[Mo_{12}Co_{5,5}Fe_{2,94}Si_{1,59}K_{0,08}O_x]_1$).

(Alternativ können die so beschickten Abschnitte 1 bis 3 auch folgende Längen aufweisen:

Abschnitt 1:  50 cm;
Abschnitt 2:  100 cm; und
Abschnitt 3:  200 cm.

Diese Längenverteilung ist günstig bei einer Mitverwendung von z. B. bis zu 50 Vol.-% Propan als inertem Verdünnungsgas).

Erfindungsgemäß günstige Ausgestaltungen eines Zweizonenrohrbündelreaktors für eine erfindungsgemäße heterogen katalysierte partielle Gasphasenoxidation von Acrolein zu Acrylsäure (z. B. als die zweite Reaktionsstufe einer zweistufigen Partialoxidation von Propylen zu Acrylsäure) können wie folgt beschaffen sein:

Alles wie beim Zweizonenrohrbündelreaktor für die erste Reaktionsstufe. Die Dicke des oberen und unteren Kontaktrohrbodens beträgt jedoch häufig 100 - 200 mm, z. B. 110 mm, oder 130 mm, oder 150 mm, oder 170 mm, oder 190 mm (in der Regel weisen beim zweistufigen Verfahren der Erst- und der Zweitstufenreaktor die gleiche Anzahl an Kontaktrohren auf).

Der Nachkühler entfällt; statt dessen münden die Kontaktrohre mit ihren unteren Öffnungen in eine am unteren Ende mit dem Behälter verbundene Haube mit Auslass für das Produktgasgemisch; die obere Temperaturzone ist die Zone A und die untere Temperaturzone ist die Temperaturzone B. Zwischen Auslass "Nachkühler" und Einlass "Reaktor für die zweite Reaktionsstufe" besteht zweckmäßig eine Zufuhrmöglichkeit für komprimierte Luft.

Die Kontaktrohr- und Thermorohrbeschickung (von oben nach unten) kann z. B. wie folgt sein:

Abschnitt 1:  20 cm Länge
Steatitringe der Geometrie 7 mm x 7 mm x 4 mm (Außendurchmesser x Länge x Innendurchmesser) als Vorschüttung. Alternativ können hier als Vorschüttung auch Steatitringe der Geometrie 7 mm x 3 mm x 4 mm verwendet werden.

Abschnitt 2:  90 cm Länge
Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschnitt 4.

Abschnitt 3: 50 cm Länge

Katalysatorbeschickung mit einem homogenen Gemisch aus 20 Gew.-% an Steatit-Ringen der Geometrie 7 mm x 3 mm x 4 mm (Außendurchmesser x Länge x Innendurchmesser) und 80 Gew.-% Schalenkatalysator aus Abschnitt 4.

Abschnitt 4: 190 cm Länge

Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: $Mo_{12}V_3W_{1,2}Cu_{2,4}O_x$).

Die Zweitstufen-Kontaktrohr- und Thermorohrbeschickung kann (von oben nach unten) auch so aussehen:

Abschnitt 1: 20 cm Länge

Steatitringe der Geometrie 7 mm x 7 mm x 4 mm (Außendurchmesser x Länge x Innendurchmesser) als Vorschüttung. Alternativ können hier als Vorschüttung auch Steatitringe der Geometrie 7 mm x 3 mm x 4 mm verwendet werden.

Abschnitt 2: 140 cm Länge

Katalysatorbeschickung mit einem homogenen Gemisch aus 25 Gew.-% an Steatit-Ringen der Geometrie 7 mm x 3 mm x 4 mm (Außendurchmesser x Länge x Innendurchmesser) und 75 Gew.-% Schalenkatalysator aus Abschnitt 3.

Abschnitt 3: 190 cm Länge

Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: $Mo_{12}V_3W_{1,2}Cu_{2,4}O_x$).

Bei Mitverwendung von z. B. bis zu 50 Vol.-% Propan als inertem Verdünnungsgas wird die Zweitstufen-Kontaktrohr- und Thermorohrbeschickung (von oben nach unten und bei Verwendung desselben Schalenkatalysators) zweckmäßig so aussehen:

Abschnitt 1: 20 cm Länge

Steatitringe der Geometrie 7 mm x 7 mm x 4 mm (oder alternativ 7 mm x 3 mm x 4 mm) (jeweils Außendurchmesser x Länge x Innendurchmesser) als Vorschüttung.

Abschnitt 2: 130 cm Länge

Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschnitt 3.

Abschnitt 3: 200 cm Länge

Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 100 46 928 (Stöchiometrie: $Mo_{12}V_3W_{1,2}Cu_{2,4}O_x$).

In den genannten Erststufenbeschickungen kann der Vollkatalysator aus Beispiel 1 der DE-A 10046957 auch ersetzt werden durch:

a) einen Katalysator gemäß Beispiel 1c aus der EP-A 15565 oder einen gemäß diesem Beispiel herzustellenden Katalysator, der jedoch die Aktivmasse $Mo_{12}Ni_{6,5}Zn_2Fe_2Bi_1P_{0,0065}K_{0,06}O_x \cdot 10\ SiO_2$ aufweist;
b) Beispiel Nr. 3 aus der DE-A 19855913 als Hohlzylindervollkatalysator der Geometrie 5 mm x 3 mm x 2 mm bzw. 5 mm x 2 mm x 2 mm;
c) Multimetalloxid II-Vollkatalysator gemäß Beispie 1 der DE-A 19746210;
d) einen der Schalenkatalysatoren 1, 2 und 3 aus der DE-A 10063162, jedoch bei gleicher Schalendicke auf Trägerringe der Geometrie 5 mm x 3 mm x 1,5 mm bzw. 7 mm x 3 mm x 1,5 mm aufgebracht.

In allen vorgenannten Zweitstufenbeschickungen kann der Schalenkatalysator gemäß Herstellungsbeispiel 5 der DE-A 10046928 ersetzt werden durch:

a) Schalenkatalysator S1 oder S7 aus der DE-A 44 42 346 mit einem Aktivmassenanteil von 27 Gew.-% und einer Schalendicke von 230 $\mu$m;
b) einem Schalenkatalysator gemäß den Beispielen 1 bis 5 aus der DE-A 198 15 281, jedoch auf Trägerringe der Geometrie 7 mm x 3 mm x 4 mm mit einem Aktivmassenanteil von 20 Gew.-% aufgebracht;
c) Schalenkatalysator mit zweiphasiger Aktivmasse der Stöchiometrie $(Mo_{10,4}V_3W_{1,2}O_x)$ $(CuMo_{0,5}W_{0,5}O_4)_{1,6}$, hergestellt gemäß DE-A 197 36 105 und mit einem Aktivmassenanteil von 20 Gew.-% auf den vorgenannten 7 mm x 3 mm x 4 mm Träger aufgebracht.

Im übrigen werden die Festbettkatalysatorschüttung 1 und die Festbettkatalysatorschüttung 2 (sowie die übrigen Verfahrensbedingungen (z. B. Zwischenregenerierung)) erfindungsgemäß zweckmäßig so gewählt (z. B. durch Verdünnung mit z. B. Inertmaterial, Mitverwendung von Inertgas), dass der Temperaturunterschied zwischen dem Heißpunktmaximum des Reaktionsgasgemisches in den einzelnen Temperaturzonen und der jeweiligen Temperatur der Temperaturzone auch im Langzeitbetrieb in der Regel 80 °C nicht überschreitet. Meist beträgt dieser Temperaturunterschied auch im Langzeitbetrieb ≤ 70 °C, häufig liegt er bei 20 bis 70 °C bzw. bis 50 °C, vorzugsweise ist dieser Temperaturunterschied auch im Langzeitbetrieb gering. Außerdem werden die Festbettkatalysatorschüttungen 1 und 2 und die sonstigen Verfahrensbedingungen aus Sicherheitsgründen in dem Fachmann an sich bekannter Weise so gewählt (z. B. durch Verdünnung mit z. B. Inertmaterial), dass die "peak-to-salt-temperature sensitivity" (die Änderung von $\Delta T^{HB}{}_A$ bzw. $\Delta T^{HB}{}_B$ bei Erhöhung der Temperatur der zugehörigen Temperaturzone um 1 °C) (vgl. Definition in der EP-A 1106598) insbesondere auch im Langzeitbetrieb ≤ 9°C, oder ≤ 7°C, oder ≤ 5°C, oder ≤ 3°C beträgt. Das vorgenannte gilt so ganz generell für den erfindungsgemäßen Langzeitbetrieb einer heterogen katalysierten partiellen Gasphasenoxidation einer organischen Ausgangsverbindung.

Nachkühler und Reaktor für die zweite Stufe sind durch ein Verbindungsrohr verbunden, dessen Länge weniger als 25 m beträgt.

In der vorstehenden Reaktoranordnung können in der zweiten Reaktionsstufe die ringförmigen Verdünnungsformkörper und die ringförmigen Katalysatorformkörper auch durch kugelförmige Verdünnungsformkörper und kugelförmige Katalysatorformkörper (jeweils mit Radius 2 bis 5 mm und mit einem Aktivmassenanteil von 10 bis 30 Gew.-%, häufig 10 bis 20 Gew.-%) ersetzt werden. Dies gilt so auch für die nachfolgenden Beispiele und Vergleichsbeispiele.

Beispiele und Vergleichsbeispiele

Ein Reaktionsrohr (V2A Stahl; 33,7 mm Außendurchmesser, 2 mm Wandstärke, 29,7 mm Innendurchmesser, Länge: 350 cm, sowie ein in der Reaktionsrohrmitte zentriertes Thermorohr (10 mm Außendurchmesser) zur Aufnahme eines Thermoelements mit dem die Temperatur im Reaktionsrohr auf seiner gesamten Länge ermittelt werden kann) wurde von oben nach unten wie folgt frisch beschickt:

Abschnitt 1:  20 cm Länge
Steatitringe der Geometrie 7 mm x 3 mm x 4 mm (Außendurchmesser x Länge x Innendurchmesser) als Vorschüttung.

Abschnitt 2:  90 cm Länge
Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschitt 4.

Abschnitt 3:  50 cm Länge
Katalysatorbeschickung mit einem homogenen Gemisch aus 20 Gew.-% an Steatit-Ringen der Geometrie 7 mm x 3 mm x 4 mm (Außendurchmesser x Länge x Innendurchmesser) und 80 Gew.-% Schalenkatalysator aus Abschnitt 4.

Abschnitt 4:  190 cm Länge
Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: $Mo_{12}V_3W_{1,2}Cu_{2,4}O_x$).

Von oben nach unten wurden die ersten 175 cm mittels eines im Gegenstrom gepumpten Salzbades A thermostatisiert das mit der Temperatur $T^A$ zugeführt wurde. Die zweiten 175 cm wurden mittels eines im Gegenstrom gepumpten Salzbades B thermostatisiert das mit der Temperatur $T^B$ zugeführt wurde.

Gasphasenoxidation:

Das vorstehend beschriebene Reaktionsrohr wurde mit einem Reaktionsgaseingangsgemisch der nachfolgenden Zusammensetzung kontinuierlich beschickt:

> 4,7 Vol.-% Acrolein,
> 0,4 Vol.-% Acrylsäure,
> 0,3 Vol.-% Propen,
> 4,7 Vol.-% molekularer Sauerstoff,
> 0,6 Vol.-% CO,
> 1,1 Vol.-% $CO_2$,
> 8,1 Vol.-% Wasser und,
> 80 Vol.-% Stickstoff.

Das Reaktionsgasgemisch durchströmte das Reaktionsrohr von oben nach unten.

Der Druck am Eingang des Reaktionsrohres betrug 2,0 atm. Die Belastung des Katalysatorfestbetts mit Acrolein betrug 140 Nl/l•h.

Die Temperaturen $T^A$, $T^B$ wurden jeweils so eingestellt, dass, bezogen auf einmaligen Durchgang des Reaktionsgasgemischs durch das Reaktionsrohr, stets ein Umsatz $U^B_{AC}$ des Acroleins von 99,5 mol-% resultierte. Von einer Zwischenregenerierung des Katalysatorfestbetts wurde abgesehen. Die nachfolgende Tabelle weist die im Langzeitbetrieb des Reaktionsrohres in Abhängigkeit von $T^A$, $T^B$ erzielte Selektivität der Acrylsäurebildung $S^{AA}$ (bezogen auf Einmaldurchgang) aus. Die angegebenen Betriebszeiten des Katalysatorfestbetts sind auf den Zeitpunkt der abgeschlossenen Formierung des Katalysatorfestbetts als Nullpunkt (Betrieb des "frisch" beschickten Katalysatorfestbetts) bezogen. $T^{maxA}$ und $T^{maxB}$ betrugen beim Betrieb des frisch formierten Katalysatorfestbetts 305 °C ($T^{maxA}$) und 297 °C ($T^{maxB}$). Die Tabelle enthält auch die im weiteren Verlauf resultierenden Werte für $T^{maxA}$ und $T^{maxB}$.

Tabelle

| Betriebszeit | $T^A$ | $T^B$ | $T^{maxA}$ | $T^{maxB}$ | $S^{AA}$ (mol-%) |
|---|---|---|---|---|---|
| 0 | 271 | 282 | 305 | 297 | 94,7 |
| 2 Monate | 271 | 281 | 305 | 297 | 94,8 |
| 4 Monate | 272 | 279 | 304 | 295 | 94,8 |
| 6 Monate | 275 | 279 | 308 | 298 | 94,8 |
| 10 Monate | 276 | 279 | 308 | 300 | 94,8 |

Wurde nach 10 monatiger Betriebsdauer $T^A$ auf 273 °C und $T^B$ auf 284 °C eingestellt konnte zwar ebenfalls ein $U^B_{AC}$ von 99,5 mol-% (bezogen auf einmaligen Durchgang) erzielt werden, $S^{AA}$ betrug jedoch lediglich 94,1 mol-%. $T^{maxA}$ betrug in diesem Fall 296 °C und $T^{maxB}$ lag bei 315 °C.

**Patentansprüche**

**1.** Verfahren zum Langzeitbetrieb einer heterogen katalysierten partiellen Gasphasenoxidation einer organischen Ausgangsverbindung zu einer organischen Zielverbindung, bei dem man ein die organische Ausgangsverbindung und molekularen Sauerstoff enthaltendes Reaktionsgaseingangsgemisch zunächst mir der Maßgabe durch ein frisch beschicktes Katalysatorfestbett, das in zwei räumlich aufeinanderfolgenden Temperaturzonen A, B aufgeschüttet ist, deren Temperaturen $T^A$ und $T^B$ so beschaffen sind, dass die Differenz $\Delta T^{BA}$ zwischen der Temperatur $T^B$ der Temperaturzonen B und der Temperatur $T^A$ der Temperaturzone A und berechnet mit der höheren der beiden Temperaturen als Minuend > 0 °C beträgt, führt, dass das Reaktionsgaseingangsgemisch die Temperaturzonen A, B in der zeitlichen Abfolge "erste A" und "dann B" durchströmt, wobei sich die Temperaturzone A bis zu einem Umsatz der organischen Ausgangsverbindung von $U^A$ = 15 bis 85 mol-% erstreckt und sich in der Temperaturzone B der Umsatz der organischen Ausgangsverbindung auf einen Wert $U^B \geq 90$ mol-% erhöht und bei dem man anschließend mit zunehmender Betriebsdauer, um der Minderung der Qualität des Katalysatorfestbetts entgegenzuwirken, die Temperatur der Temperaturzonen A, B verändert, **dadurch gekennzeichnet, dass** man mit zunehmender Betriebsdauer die Temperatur derjenigen Temperaturzone, die zunächst die tiefere Temperatur aufwies, erhöht, und die Differenz $\Delta T^{BA}$ zwischen den Temperaturen der beiden Temperaturzonen verringert, wobei bei der Differenzbildung die Temperatur derjenigen Temperaturzone, die zunächst die höhere Temperatur aufwies, ihre Position als Minuend beibehält.

2. Verfahren nach Anspruch 1, **dadurch gekennzeichnet, dass** die partielle Gasphasenoxidation diejenige von Propylen zu Acrolein und/oder Acrylsäure, oder diejenige von iso-Buten zu Methacrolein und/oder Methacrylsäure, oder diejenige von Acrolein zu Acrylsäure, oder diejenige von Methacrolein zu Methacrylsäure, oder diejenige von Propan zu Acrylsäure, oder diejenige von iso-Butan zu Methacrylsäure ist.

3. Verfahren nach Anspruch 1, **dadurch gekennzeichnet, dass** die organische Ausgangsverbindung eine organische Verbindung aus der Gruppe umfassend Propylen, Acrolein, 1-Buten, 2-Buten, Ethan, Benzol, m-Xylol, p-Xylol, iso-Butan, iso-Buten, tert.-Butanol, iso-Butyraldehyd, Methylether des tert.-Butanols, o-Xylol, Naphtalin, Butadien, Ethylen, Propan und Methacrolein ist.

4. Verfahren nach Anspruch 1, **dadurch gekennzeichnet, dass** die Gasphasenoxidation die Partialoxidation von Acrolein zu Acrylsäure in einer zweistufigen Gasphasen-Partialoxidation von Propylen zu Acrylsäure ist.

5. Verfahren nach einem der Ansprüche 1 bis 4, **dadurch gekennzeichnet, dass** die Temperaturzone A die zunächst tiefere Temperatur aufwies.

6. Verfahren nach Anspruch 5, **dadurch gekennzeichnet, dass** mit zunehmender Betriebsdauer die Temperatur der Temperaturzone B erniedrigt wird.

7. Verfahren nach Anspruch 5, **dadurch gekennzeichnet, dass** mit zunehmender Betriebsdauer die Temperatur der Temperaturzone B erhöht wird.

8. Verfahren nach einem der Ansprüche 1 bis 7, **dadurch gekennzeichnet, dass** $\Delta T^{BA}$ innerhalb einer Betriebsdauer von 12 Monaten sein Vorzeichen nicht ändert.

9. Verfahren nach einem der Ansprüche 1 bis 8, **dadurch gekennzeichnet, dass** der Betrag von $\Delta T^{BA}$ 50 °C nicht überschreitet.

10. Verfahren nach einem der Ansprüche 1 bis 9, **dadurch gekennzeichnet, dass** der Unterschied zwischen der maximalen Reaktionstemperatur in der Temperaturzone A, $T^{maxA}$, und der maximalen Reaktionstemperatur in der Temperaturzone B, $\mathbf{T}^{maxB}$, gebildet als $\mathbf{T}^{maxA} - \mathbf{T}^{maxB}$ sowohl zu Beginn des Verfahrens, als auch im Langzeitbetrieb $\geq 0$ °C beträgt.

11. Verfahren nach einem der Ansprüche 1 bis 10, **dadurch gekennzeichnet, dass** es eine Partialoxidation von Acrolein zu Acrylsäure ist, und $U^{A}$ 40 bis 85 mol-% beträgt.

12. Verfahren nach einem der Ansprüche 1 bis 11, **dadurch gekennzeichnet, dass** es eine Partialoxidation von Acrolein zu Acrylsäure ist, und sowohl die Temperatur der Temperaturzone A als auch der Temperaturzone B im Langzeitbetrieb 230 bis 340 °C beträgt.

13. Verfahren nach einem der Ansprüche 1 bis 12, **dadurch gekennzeichnet, dass** es eine Partialoxidation von Acrolein zu Acrylsäure ist, und die Belastung des Katalysatorfestbetts mit Acrolein $\geq 90$ Nl/l•h und $\leq 300$ Nl/l•h beträgt.

14. Verfahren nach einem der Ansprüche 1 bis 13, **dadurch gekennzeichnet, dass** es eine Partialoxidation von Acrolein zu Acrylsäure ist, und der Unterschied zwischen der maximalen Reaktionstemperatur in der Temperaturzone A, $T^{maxA}$, und der maximalen Reaktionstemperatur in der Temperaturzone B, $T^{maxB}$, gebildet als $T^{maxA} - T^{maxB}$ sowohl zu Beginn des Verfahrens, als auch im Langzeitbetrieb $\geq 0$ °C und $\leq 15$ °C beträgt.

15. Verfahren nach einem der Ansprüche 1 bis 10, **dadurch gekennzeichnet, dass** es eine Partialoxidation von Propylen zu Acrolein ist, und $U^{A}$ 30 bis 80 mol-% beträgt.

**16.** Verfahren nach einem der Ansprüche 1 bis 10 oder nach Anspruch 15, **dadurch gekennzeichnet, dass** es eine Partialoxidation von Propylen zu Acrolein ist, und sowohl die Temperatur der Temperaturzone A als auch der Temperaturzone B im Langzeitbetrieb 290 bis 380 °C beträgt.

**17.** Verfahren nach einem der Ansprüche 1 bis 10 oder nach einem der Ansprüche 15 und 16, **dadurch gekennzeichnet, dass** es eine Partialoxidation von Propylen zu Acrolein ist, und die Belastung des Katalysatorfestbetts mit Propylen ≥ 120 Nl/l•h und ≤ 300 Nl/l•h beträgt.

**18.** Verfahren nach einem der Ansprüche 1 bis 17, **dadurch gekennzeichnet, dass** es in einem Vielkontaktrohr-Festbettreaktor durchgeführt wird.

**EUROPÄISCHER RECHERCHENBERICHT**

**Nummer der Anmeldung**

EP 06 10 0535

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | DE 199 48 241 A1 (BASF AG) 12. April 2001 (2001-04-12) * Ansprüche 1-27; Tabelle 2 * ----- | 1-18 | INV. C07C57/05 |
| | | | **RECHERCHIERTE SACHGEBIETE (IPC)** |
| | | | C07C |

Der vorliegende Recherchenbericht wurde für alle Patentansprüche erstellt

| Recherchenort | Abschlußdatum der Recherche | Prüfer |
|---|---|---|
| München | 20. Juli 2006 | Kleidernigg, O |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes
   Dokument

**ANHANG ZUM EUROPÄISCHEN RECHERCHENBERICHT
ÜBER DIE EUROPÄISCHE PATENTANMELDUNG NR.**

EP 06 10 0535

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-07-2006

| Im Recherchenbericht angeführtes Patentdokument | Datum der Veröffentlichung | Mitglied(er) der Patentfamilie | Datum der Veröffentlichung |
|---|---|---|---|
| DE 19948241 A1 | 12-04-2001 | KEINE | |

Für nähere Einzelheiten zu diesem Anhang : siehe Amtsblatt des Europäischen Patentamts, Nr.12/82

EPO FORM P0461

## EP 1 734 030 A1

### IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2351151 A
- DE 2526238 A
- EP 092097 A
- EP 058927 A
- DE 4132263 A
- DE 4132684 A
- DE 4022212 A
- EP 522871 A
- GB 1464198 A
- GB 1291354 A
- DE 2025430 A
- DE 1254137 B
- DE 2159346 A
- EP 372972 A
- WO 8907101 A
- DE 4311608 A
- DE 10131297 A
- EP 1090684 A
- EP 608838 A
- DE 10046672 A
- EP 529853 A
- WO 0196270 A
- DE 10028582 A
- WO 2004009525 A
- WO 2005113127 A
- DE 10046957 A
- EP 1097745 A
- DE 4431957 A
- DE 10046928 A
- DE 19910506 A
- DE 19622331 A
- DE 10121592 A
- EP 700714 A
- DE 19910508 A
- EP 415347 A
- EP 471853 A
- EP 700893 A
- DE 19927624 A
- DE 19948523 A
- WO 0053557 A
- DE 19948248 A
- WO 0053558 A
- WO 2004085365 A
- WO 2004085363 A
- WO 2004085367 A
- WO 2004085369 A
- WO 2004085370 A
- WO 2004085362 A

- EP 1159247 A
- EP 1159246 A
- EP 1159248 A
- EP 1106598 A
- WO 2005021149 A
- US 20050049435 A
- WO 2004007064 A
- WO 05063673 A
- WO 05063674 A
- DE 19948241 A
- DE 2830765 C
- DE 2513405 C
- US 3147084 A
- DE 2201528 A
- EP 383224 A
- DE 2903218 A
- EP 253409 A
- EP 1180508 A
- DE 19902562 A
- DE 10351269 A
- DE 10350812 A
- DE 10350822 A
- EP 614872 A
- DE 102004008573 A
- WO 05082517 A
- DE 102004025445 A
- DE 10232748 A
- DE 19955176 A
- DE 10101695 A
- DE 19955168 A
- DE 10063162 A
- DE 3338380 C
- EP 15565 A
- DE 2380765 C
- EP 807465 A
- EP 279374 A
- DE 3300044 A
- EP 575897 A
- US 4438217 A
- DE 19855913 A
- WO 9824746 A
- DE 19746210 A
- JP 3294239 A
- EP 293224 A
- WO 02062737 A
- DE 4023239 A
- DE 2909671 A
- EP 293859 A
- EP 714700 A

- DE 19815281 A
- DE 4335973 A
- EP 668104 A
- DE 19736105 A
- DE 19740493 A
- DE 19528646 A
- DE 4442346 A
- EP 468290 B
- DE 2201528 B
- EP 382098 A
- WO 0136364 A
- DE 10302715 A
- DE 10261186 A
- EP 982287 A
- EP 982289 A
- DE 19924532 A
- DE 10115277 A
- DE 19606877 A
- DE 19740252 A
- DE 19627847 A
- DE 10053086 A
- EP 982288 A
- EP 20219277 A
- EP 200219278 A
- EP 20219279 A
- EP 0214187 W
- EP 0214188 W
- EP 0214189 W
- EP 873783 A
- EP 1270065 A